# FASCICULE DE BREVET EUROPEEN

(11) **EP 2 039 724 B1**
(45) Date de publication et mention de la délivrance du brevet: **03.08.2011**
(21) Numéro de dépôt: 08164634.1
(22) Date de dépôt: 18.09.2008
(51) Int. Cl.: C09B 23/04, A61Q 5/10, A61K 8/49

(54) **Colorant dérivé d'indole styryle à linker alkylène, composition tinctoriale comprenant ce colorant, procédé d'éclaircissement des matières kératiniques à partir de ce colorant**
Indol-Styryl Farbstoffe mit Alkylen-Brückenglied, diese enthaltende Färbepräparate sowie Verfahren zum Aufhellen keratinischer Materialien unter Verwendung derartiger Farbstoffe
Indole-derived styryl dye comprising an alkylene linker, dye composition comprising this dye, process for lightening keratin materials using this dye

(30) Priorité: 21.09.2007 FR 0757773
(43) Date de publication de la demande: 25.03.2009
(73) Titulaire: L'Oréal, 75008 Paris (FR)
(72) Inventeur: Greaves, Andrew, 77700 Bailly Romainvilliers (FR); Daubresse, Nicolas, 78170 La Celles St Cloud (FR)
(74) Mandataire: Fevrier, Murielle Françoise E.

(56) Documents cités:
- EP-A- 1 792 605
- US-A1- 2003 176 316

## Description

L'invention concerne la coloration de matières kératiniques à l'aide de colorants dérivés d'indole styryle à linker alkylène.
Il est connu de teindre les fibres kératiniques, notamment humaines, par une coloration directe. Le procédé classiquement utilisé en coloration directe consiste à appliquer sur les fibres kératiniques des colorants directs qui sont des molécules colorées et colorantes ayant une affinité pour les fibres, à les laisser diffuser, puis à rincer les fibres.
Les colorants directs qui sont classiquement utilisés sont par exemple des colorants du type nitrés benzéniques, des colorants anthraquinoniques, des nitropyridines, des colorants du type azoïque, xanthénique, acridinique, azinique ou triarylméthane.
Par ailleurs, la coloration des fibres kératiniques à partir de ces colorants directs classiques ne permet pas d'éclaircir de façon notable les fibres kératiniques.
L'éclaircissement de la couleur de fibres kératiniques, plus particulièrement foncées vers des nuances plus claires, en modifiant éventuellement la nuance de celles-ci, constitue une demande importante.
Classiquement, pour obtenir une coloration plus claire, on met en oeuvre un procédé de décoloration chimique. Ce procédé consiste à traiter les fibres kératiniques, notamment les cheveux, par un système oxydant fort, généralement constitué par du peroxyde d'hydrogène associé ou non à des persels, le plus souvent en milieu alcalin.
Ce système de décoloration présente l'inconvénient de dégrader les fibres kératiniques, notamment les cheveux et d'altérer leurs propriétés cosmétiques. Les fibres ont en effet tendance à devenir rêches, plus difficilement démêlables et plus fragiles. Enfin, l'éclaircissement ou la décoloration de fibres kératiniques par des agents oxydants est incompatible avec les traitements de modification de la forme desdites fibres particulièrement dans les traitements de défrisage.
Une autre technique d'éclaircissement consiste à appliquer sur les cheveux foncés des colorants directs fluorescents. Cette technique décrite notamment dans les documents WO 03/028685, WO 2004/091473, EP 1792605 permet de respecter la qualité de la fibre kératinique lors du traitement. Cependant certains de ces colorants directs fluorescents ne possèdent pas des performances satisfaisantes notamment en termes de montée de couleur dans la fibre kératinique, de sélectivité, de stabilité, de ténacité et de solubilité dans les milieux de teintures cosmétiques.

Le but de la présente invention est de fournir de nouveaux systèmes de coloration de matières kératiniques en particulier les fibres kératiniques humaines, notamment les cheveux, qui ne présentent pas les inconvénients des procédés de décoloration existants.
Particulièrement, un des buts de la présente invention est de colorer de façon chromatique et rémanente vis-à-vis des agressions extérieures les matières kératiniques. Un autre but de la présente invention est de fournir des systèmes de coloration directe permettant d'obtenir des effets éclaircissants notamment sur des fibres kératiniques naturellement ou artificiellement foncées, tenaces face à des shampooings successifs, qui ne dégradent pas les fibres kératiniques et qui n'altèrent pas leurs propriétés cosmétiques.

Ce but est atteint avec la présente invention qui a pour objet un procédé de coloration de matières kératiniques, en particulier les fibres kératiniques, notamment humaines telles que les cheveux, plus particulièrement les cheveux foncés, consistant à appliquer sur les matières kératiniques, une composition tinctoriale, comprenant dans un milieu cosmétique approprié, au moins un colorant dérivé d'indole styryle à linker alkyle ou alkylène, choisi parmi les colorants de formule **(I)** : 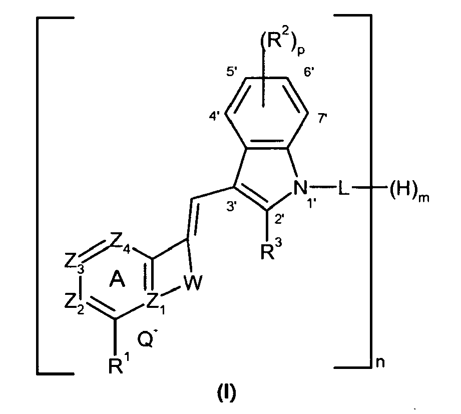 leurs sels d'acide organique ou minéral, isomères optiques, isomères géométriques, et les solvates tels que hydrates ;
formule **(I)** dans laquelle :
➢ **n** représente 1 ou 2 ;
➢ **m** représente 0 ou 1 ;
➢ **p** représente un entier compris inclusivement entre 0 et 4 ;
➢ **Q**⁻ représente un contre-ion anionique ;
➢ **W** représente une chaîne (C₂-C₃)alkylène ou (C₂-C₃)alkénylène éventuellement substituée par un ou plusieurs groupement (C₁-C₄)alkyle ou aryle ; particulièrement W représente un chaîne (C₂-C₃)alkylène non substituée ;
➢ **Z₁** représente un atome de carbone ou d'azote quaternisé N⁺ ;
➢ **Z₂**, **Z₃**, **Z₄**, identiques ou différents, représentent un atome d'azote, un groupement CR⁴ ou N⁺R⁵ ; étant entendu qu'un seul atome d'azote quaternisé N⁺ ou N⁺R⁵ ne peut être présent dans le cycle aromatique A ;
➢ **R¹**, **R²** et **R⁴,** identiques ou différents, représentent un atome d'halogène, un groupement (di)(C₁-C₆)(alkyl)amino, cyano, hydroxy, (poly)halogéno(C₁-C₆)alkyle tel que trifluorométhyle, acyl(C₁-C₄)(alkyl)amino, (C₁-C₆)alcoxy, (C₁-C₆)alkylthio, (poly)hydroxy(C₂-C₆)alcoxy, (C₁-C₆)alkylcarbonyloxy, (C₁-C₆)alcoxycarbonyle, (C₁-C₆)alkylcarbonylamino, (di)(C₁-C₄)(alkyl)aminocarbonyle, (C₁-C₆)alkylsulfonyl(C₁-C₄)(alkyl)amino, (di)(C₁-C₄)(alkyl)aminosulfonyle, ou un groupement (C₁-C₆)alkyle éventuellement substitué par un groupement choisi parmi (C₁-C₆)alcoxy, hydroxy, (di)(C₁-C₆)(alkyl)amino,; ou alors lorsque p est une entier supérieur ou égal à 2, deux radicaux R² contigus forment ensemble avec l'atome de carbone qui les portent un cycle benzo ;
➢ **R³** représente un atome d'hydrogène, un groupement (C₁-C₆)alkyle éventuellement substitué, (di)(C₁-C₆)(alkyl)amino, aryle tel que phényle, hétérocycloalkyle éventuellement substitué tel que morpholino, pipéridino, pipérazino éventuellement substitué par un groupe (C₁-C₃)alkyle ;
➢ **R⁵** représente un groupement (C₁-C₆)alkyle éventuellement substitué, ou un aryl(C₁-C₆)alkyle ; particulièrement R⁵ représente un groupement (C₁-C₄)alkyle tel que le méthyle ou l'éthyle ;
➢ **L** représente un liaison σ ou une chaîne hydrocarbonée bivalente en C₁-C₂₀, éventuellement substituée, éventuellement interrompue i) par un ou plusieurs groupes divalents ou leurs combinaisons choisis parmi : -N(R)- ; -N⁺(R)(R°)-, An⁻; -O-; -C(O)- et -S(O)₂- avec R, R°, identiques ou différents, choisi parmi un hydrogène, un radical (C₁-C₄)alkyle, hydroxy(C₁-C₄)alkyle, amino(C₁-C₄)alkyle et An⁻ représentant un contre-ion anionique ou ii) par un hétérocycle cationique ou hétéroaryle cationique Hét⁺, An⁻, avec An⁻ tel que défini précédemment et Het⁺ représentant un hétérocycle comprenant de 5 à 10 chaînons, saturé ou non, ou un hétéroaryle comprenant de 5 à 10 chaînons tel que imidazolium, pyridinium, pipérazinium, pipéridinium, benzoimidazolium; notamment L représente une chaîne (C₂-C₆)alkylène telle que éthylène, propylène ou butylène ;
étant entendu que lorsque n vaut 2 alors m vaut zéro, et lorsque n vaut 1 alors m vaut 1.

Un autre objet de l'invention est une composition tinctoriale pour la coloration de matières kératiniques, en particulier de fibres kératiniques telles que les cheveux comprenant, dans un milieu cosmétique, au moins un colorant dérivé d'indole styryle à linker alkyle ou alkylène de formule **(I)** tel que défini précédemment.

L'invention a aussi pour objet de nouveaux colorants dérivés d'indole styryle à linker alkyle ou alkylène de formule **(I)** tels que définis précédemment.

Le procédé de coloration selon l'invention permet de colorer de façon visible les matières kératiniques foncées, notamment les cheveux foncés. Ce procédé permet également de colorer des fibres kératiniques décolorées de façon puissante, peu sélective, homogène et chromatique. De plus le procédé de l'invention permet d'obtenir une coloration des matières kératiniques sans dégrader ladite matière, rémanente vis-à-vis de shampooings, des agressions courantes (soleil, transpiration), et des autres traitements capillaires. Ces colorants par ailleurs, étendent la gamme colorielle aux jaunes et rouges. Le procédé de l'invention permet également d'obtenir un éclaircissement des matières kératiniques particulièrement foncées.

Au sens de l'invention, on entend par matière kératinique foncée celle qui présente une luminescence L* chiffrée dans le système C.I.E.L*a*b*, inférieure ou égale à 45 et de préférence inférieure ou égale à 40, sachant par ailleurs que L*=0 équivaut au noir et L*=100 au blanc.
Au sens de l'invention, on entend par cheveux naturellement ou artificiellement foncés, des cheveux dont la hauteur de ton est inférieure ou égale à 6 (blond foncé) et de préférence inférieure ou égale à 4 (châtain).
L'éclaircissement des cheveux est évalué par la variation de «hauteur de ton» avant et après application du composé de formule **(I)**. La notion « ton » repose sur la classification des nuances naturelles, un ton séparant chaque nuance de celle qui la suit ou la précède immédiatement. Cette définition et la classification des nuances naturelles est bien connue des professionnels de la coiffure et publiée dans l'ouvrage « Science des traitements capillaires » de Charles ZVIAK 1988, Ed. Masson, p. 215 et 278.
Les hauteurs de ton s'échelonnent de 1 (noir) à 10 (blond très clair), une unité correspondant à un ton ; plus le chiffre est élevé et plus la nuance est claire.
Un cheveu coloré artificiellement est un cheveu dont la couleur a été modifiée par un traitement de coloration par exemple une coloration avec des colorants directs ou des colorants d'oxydation.
Au sens de l'invention, on entend par « cheveux décolorés », des cheveux dont la hauteur de ton est supérieure à 6 et de préférence supérieure à 7.
De préférence, la composition doit, après application sur des cheveux, par exemple châtains, amener aux résultats ci-dessous.
- On s'intéresse aux performances de réflectance des cheveux lorsqu'on les irradie avec de la lumière visible dans la gamme de longueurs d'onde allant de 400 à 700 nanomètres.
- On compare alors les courbes de réflectance en fonction de la longueur d'onde, des cheveux traités avec la composition de l'invention et des cheveux non traités.
- La courbe correspondant aux cheveux traités doit montrer une réflectance dans la gamme des longueurs d'onde allant de 500 à 700 nanomètres supérieure à la courbe correspondant aux cheveux non traités.
- Cela signifie que, dans la gamme de longueur d'onde allant de 540 à 700 nanomètres, il existe au moins une plage où la courbe de réflectance correspondant aux cheveux traités est supérieure à la courbe de réflectance correspondant aux cheveux non traités. On entend par "supérieure", un écart d'au moins 0,05% de réflectance, et de préférence d'au moins 0,1%. Ceci n'empêche pas qu'il peut exister dans la gamme de longueur d'onde allant de 540 à 700 nanomètres, au moins une plage où la courbe de réflectance correspondant aux cheveux traités soit superposable, ou inférieure à la courbe de réflectance correspondant aux cheveux non traités.

De préférence, la longueur d'onde où l'écart est maximal entre la courbe de réflectance des cheveux traités et celle des cheveux non traités, se situe dans la gamme de longueur d'onde allant de 500 à 650 nanomètres, et de préférence dans la gamme de longueur d'onde allant de 550 à 620 nanomètres.

Au sens de la présente invention, et à moins qu'une indication différente ne soit donnée :
- les radicaux « aryle » ou « hétéroaryle » ou « benzo » ou la partie aryle ou hétéroaryle d'un radical peuvent être substitués par au moins un substituant choisi parmi :
   - un radical alkyle en C₁-C₁₆, de préférence en C₁-C₈, éventuellement substitué par un ou plusieurs radicaux choisis parmi les radicaux hydroxy, alcoxy en C₁-C₂, (poly)-hydroxyalcoxy en C₂-C₄, acylamino, amino substitué par deux radicaux alkyle, identiques ou différents, en C₁-C₄, éventuellement porteurs d'au moins un groupement hydroxy ou, les deux radicaux pouvant former avec l'atome d'azote auquel ils sont rattachés, un hétérocycle comprenant de 5 à 7 chaînons, de préférence de 5 ou 6 chaînons, saturé ou insaturé éventuellement substitué comprenant éventuellement un autre hétéroatome identique ou différent de l'azote ;
   - un atome d'halogène tel que chlore, fluor ou brome ;
   - un groupement hydroxy ;
   - un radical alcoxy en C₁-C₂ ;
   - un radical alkylthio en C₁-C₂ ;
   - un radical (poly)-hydroxyalcoxy en C₂-C₄ ;
   - un radical amino ;
   - un radical héterocycloalkyle à 5 ou 6 chaînons ;
   - un radical hétéroaryle à 5 ou 6 chaînons éventuellement cationique, préférentiellement imidazolium, et éventuellement substitué par un radical (C₁-C₄) alkyle, préférentiellement méthyle ;
   - un radical amino substitué par un ou deux radicaux alkyle, identiques ou différents, en C₁-C₆ éventuellement porteurs d'au moins :
      i) un groupement hydroxy,
      ii) un groupement amino éventuellement substituté par un ou deux radicaux alkyle en C₁-C₃ éventuellement substitués, lesdits radicaux alkyle pouvant former avec l'atome d'azote auquel ils sont rattachés, un hétérocycle comprenant de 5 à 7 chaînons, saturé ou insaturé éventuellement substitué comprenant éventuellement au moins un autre hétéroatome différent ou non de l'azote,
   - -N(R)-C(O)-R' dans lequel le radical R est un atome d'hydrogène, un radical alkyle en C₁-C₄ éventuellement porteur d'au moins un groupement hydroxy et le radical R' est un radical alkyle en C₁-C₂ ;
   - R₂N-C(O)- dans lequel les radicaux R, identiques ou non, représentent un atome d'hydrogène, un radical alkyle en C₁-C₄ éventuellement porteur d'au moins un groupement hydroxy ;
   - R'S(O)₂-N(R)- dans lequel le radical R représente un atome d'hydrogène, un radical alkyle en C₁-C₄ éventuellement porteur d'au moins un groupement hydroxy et le radical R' représente un radical alkyle en C₁-C₄, un radical phényle ;
   - R₂N-S(O)₂- dans lequel les radicaux R, identiques ou non, représentent un atome d'hydrogène, un radical alkyle en C₁-C₄ éventuellement porteur d'au moins un groupement hydroxy,
   - un radical carboxylique sous forme acide ou salifiée (de préférence avec un métal alcalin ou un ammonium, substitué ou non) ;
   - un groupement cyano ;
   - un groupement polyhalogénoalkyle comprenant de 1 à 6 atomes de carbones et de 1 à 6 atome d'halogène, identiques ou différents, le groupement polyhalogénoalkyle est par exemple le trifluorométhyle ;
- la partie hétérocyclique d'un radical non aromatique peut être substituée par au moins un substituant choisi parmi les groupements :
   - hydroxy,
   - alcoxy en C₁-C₄,
   - alkyle en C₁-C₄,
   - (poly)hydroxyalcoxy en C₂-C₄,
   - un radical alkylthio en C₁-C₂;
   - RC(O)-N(R')- dans lequel le radical R' est un atome d'hydrogène, un radical alkyle en C₁-C₄ éventuellement porteur d'au moins un groupement hydroxy et le radical R est un radical alkyle en C₁-C₂, amino substitué par deux groupements alkyle identiques ou différents en C₁-C₄, éventuellement porteurs d'au moins un groupement hydroxy ;
   - RC(O)-O- dans lequel le radical R est un radical alkyle en C₁-C₄, amino substitué par un ou deux groupements alkyle identiques ou différents en C₁-C₄ éventuellement porteurs d'au moins un groupement hydroxy, lesdits radicaux alkyle pouvant former avec l'atome d'azote auquel ils sont rattachés, un hétérocycle comprenant de 5 à 7 chaînons, saturé ou insaturé éventuellement substitué comprenant éventuellement au moins un autre hétéroatome différent ou non de l'azote ;
   - RO-C(O)- dans lequel le radical R est un radical alkyle en C₁-C₄ éventuellement porteurs d'au moins un groupement hydroxy ;
- un hétérocyclique, ou une partie non aromatique d'un radical aryle ou hétéroaryle, peut également être substitué par un ou plusieurs groupements oxo ou thioxo ;
- un radical « aryle » représente un groupement mono ou polycyclique, condensé ou non, comprenant de 6 à 22 atomes de carbones, et dont au moins un cycle est aromatique ; préférentiellement le radical aryle est un phényle, biphényle, naphtyle, indényle, anthracényle, ou tétrahydronaphtyle ;
- un « radical hétéroaryle » représente un groupement mono ou polycyclique, condensé ou non, éventuellement cationique, comprenant de 5 à 22 chaînons, de 1 à 6 hétéroatomes choisis parmi l'atome d'azote, d'oxygène, de soufre et de sélénium, et dont au moins un cycle est aromatique ; préférentiellement un radical hétéroaryle est choisis parmi acridinyle, benzimidazolyle, benzobistriazolyle, benzopyrazolyle, benzo-pyridazinyle, benzoquinolyle, benzothiazolyle, benzotriazolyle, benzoxazolyle, pyridinyle, tétrazolyle, dihydrothiazolyle, imidazopyridinyle, imidazolyle, indolyle, isoquinolyle, naphthoimidazolyle, naphthooxazolyle, naphthopyrazolyle, oxadiazolyle, oxazolyle, oxazolopyridyle, phénazinyle, phénooxazolyle, pyrazinyle, pyrazolyle, pyrilyle, pyrazoyltriazyle, pyridyle, pyridinoimidazolyle, pyrrolyle, quinolyle, tétrazolyle, thiadiazolyle, thiazolyle, thiazolopyridinyle, thiazoylimidazolyle, thiopyrylyle, triazolyle, xanthylyle et son sel d'ammonium ;
- un « radical hétérocyclique ou hétérocycle» est un radical non aromatique mono ou polycyclique, condensé ou non, contenant de 5 à 22 chaînons, comportant de 1 à 6 hétéroatomes choisis parmi l'atome d'azote, d'oxygène, de soufre et de sélénium tel que pyrrolidine, morpholinyle, pipéridinyle ou pipérazinyle, ;
- un « radical alkyle » est un radical hydrocarboné en C₁-C₁₆, linéaire ou ramifié, de préférence en C₁-C₈ ;
- l'expression « éventuellement substitué » attribué au radical alkyle sous entend que ledit radical alkyle peut être substitué par un ou plusieurs radicaux choisis parmi les radicaux i) hydroxy, ii) alcoxy en C₁-C₄, iii) acylamino, iv) amino éventuellement substitué par un ou deux radicaux alkyle, identiques ou différents, en C₁-C₄, lesdits radicaux alkyles pouvant former avec l'atome d'azote qui les portent un hétérocyle comprenant de 5 à 7 chaînons, comprenant éventuellement un autre hétéroatome différent ou non de l'azote ; v) ou un groupement ammonium quaternaire -N⁺R'R''R''', M⁻ pour lequel R', R'', R"', identiques ou différents représentent un atome d'hydrogène, ou un groupement alkyle en C₁-C₄, ou alors -N⁺R'R''R''' forme un hétéroaryle tel que imidazolium éventuellement substitué par un groupement C₁-C₄ alkyle, et M⁻ représente le contre-ion de l'acide organique, minéral ou de l'halogénure correspondant,
- un « radical alcoxy » est un radical alkyle-oxy ou alkyl-O- pour lequel le radical alkyle est un radical hydrocarboné, linéaire ou ramifié, en C₁-C₁₆ préférentiellement en C₁-C₈;
- un « radical alkylthio » est un radical alkyl-S- pour lequel le radical alkyle est un radical hydrocarboné, linéaire ou ramifié, en C₁-C₁₆ préférentiellement en C₁-C₈,;
- une « chaîne alkylène » représente un chaîne divalente ; particulièrement en C₁-C₆, plus particulièrement en C₁-C₃ lorsque la chaîne est linéaire ; éventuellement substituée par un ou plusieurs, identiques ou différents, atomes d'halogènes ou groupements choisi parmi hydroxy, alkoxy, (di)(alkyl)amino, R^{a}-Z^{a}-C(Z^{b})- avec Z^{a}, Z^{b}, identiques ou différents, représentant un atome d'oxygène, de soufre, ou un groupement NR^{a}', et R^{a}, représentant un métal alcalin, un atome d'hydrogène, ou un groupement alkyle et R^{a}' représentant un atome d'hydrogène ou un groupement alkyle ;
- une « chaîne alkénylène » représente un chaîne divalente ; particulièrement en C₂-C₆, plus particulièrement -C=C-, comprenant de 1 à 3 doubles liaisons π, conjuguées
ou non, éventuellement substituée par les mêmes groupes que pour la chaîne alkylène ;
- une « chaîne hydrocarbonée en C₁-C₂₀, saturée ou insaturée, éventuellement substituée », représente une chaîne hydrocarbonée, particulièrement an C₁-C₈, comprenant éventuellement de 1 à 3 doubles liaisons π, conjuguée ou non, particulièrement la chaîne hydrocarbonée est saturée ; ladite chaîne est éventuellement substituée par un ou plusieurs, identiques ou différents, atomes d'halogènes ou groupements choisi parmi hydroxy, alkoxy, (di)(alkyl)amino, R^{b}-Z^{b}-C(Z^{c})- avec Z^{b}, Z^{c}, identiques ou différents, représentant un atome d'oxygène, de soufre, ou un groupement NR^{b}', et R^{b}, représentant un métal alcalin, un atome d'hydrogène, ou un groupement alkyle et R^{b}' représentant un atome d'hydrogène ou un groupement alkyle ;
- les bornes délimitant l'étendue d'une plage de valeurs sont comprises dans cette plage de valeurs ;
- un « sel d'acide organique ou minéral » est plus particulièrement choisi parmi un sel dérivé i) d'acide chlorhydrique HCl, ii) d'acide bromhydrique HBr, iii) d'acide sulfurique H₂SO₄, iv) d'acides alkylsulfoniques : Alk-S(O)₂OH tels que d'acide méthylsulfonique et d'acide éthylsulfonique ; v) d'acides arylsulfoniques : Ar-S(O)₂OH tel que d'acide benzène sulfonique et d'acide toluène sulfonique ; vi) d'acide citrique ; vii) d'acide succinique ; viii) d'acide tartrique ; ix) d'acide lactique, x) d'acides alcoxysulfiniques : Alk-O-S(O)OH tels que d'acide méthoxysulfinique et d'acide éthoxysulfinique ; xi) d'acides aryloxysulfiniques tels que d'acide toluèneoxysulfinique et d'acide phénoxysulfinique ; xii) d'acide phosphorique H₃PO₄; xiii) d'acide acétique CH₃C(O)OH ; xiv) d'acide triflique CF₃SO₃H et xv) d'acide tétrafluoroborique HBF4;
- un « contre-ion anionique » est un anion ou un groupement anionique associé à la charge cationique du colorant ; plus particulièrement le contre-ion anionique est choisi parmi i) les halogénures tels que le chlorure, le bromure ; ii) les nitrates ; iii) les sulfonates parmi lesquels les C₁-C₆ alkylsulfonates : Alk-S(O)₂O⁻ tels que le méthylsulfonate ou mésylate et l'éthylsulfonate ; iv) les arylsulfonates : Ar-S(O)₂O⁻ tel que le benzènesulfonate et le toluènesulfonate ou tosylate ; v) le citrate ; vi) le succinate ; vii) le tartrate ; viii) le lactate ; ix) les alkylsulfates : Alk-O-S(O)O⁻ tels que le méthysulfate et l'éthylsulfate ; x) les arylsulfates : Ar-O-S(O)O⁻ tels que le benzènesulfate et le toluènesulfate ; xi) les alcoxysulfates : Alk-O-S(O)₂O⁻ tel que le méthoxy sulfate et l'éthoxysulfate ; xii) les aryloxysulfates : Ar-O-S(O)₂O⁻, xiii) le phosphate ; xiv) l'acétate ; xv) le triflate ; et xvi) les borates tels que le tétrafluoroborate ;
- les « solvates » représentent les hydrates ainsi que l'association avec des alcools linéaires ou ramifiés en C₁-C₄ linéaire ou ramifié tels que l'éthanol, l'isopropanol, le n-propanol.

Les colorants dérivés d'indole styryle à linker alkyle ou alkylène de formule **(I)** tels que définis précédemment sont des colorants capables de fluorescer, *i.e.* capables d'absorber dans le rayonnement UV ou visible à une longueur d'onde λ_{abs} comprise entre 250 et 800 nm et capables de réémettre dans le domaine du visible à une longueur d'onde d'émission λ_{ém} comprise entre 400 et 800 nm.

De préférence les composés fluorescents de formules **(I)** de l'invention sont des colorants capables d'absorber dans le visible λ_{abs} comprise entre 400 et 800 nm et de réémettre dans le visible λ_{ém} comprise entre 400 et 800 nm. Plus préférentiellement les colorants de formules **(I)** sont des colorants capables d'absorber à une λ_{abs} comprise entre 400 nm et 550 nm et de réémettre dans le visible à une λ_{ém} comprise entre 450 et 600 nm.

Un mode particulier concerne les colorants fluorescents de formule **(I)** avec n et m qui valent 1.

Selon un autre mode particulier de l'invention les colorants fluorescents de formule **(I)** sont des colorants fluorescents avec n = 2 et m = 0.

Un aspect particulier de l'invention concerne les colorants fluorescent de formule **(I)** comprenant un motif A dans lequel :
a) soit **Z₁** représente un atome d'azote quaternisé N⁺, et **Z₂**, **Z₃** et **Z₄** représentent CR⁴, avec **R⁴** étant plus particulièrement un atome d'hydrogène ou un groupement (C₁-C₄)alkyle, ou **Z₃** et **Z₄** représentent CH et **Z₂** représente un groupement (C₁-C₆)alkylcarbonylamino ou (di)(C₁-C₄)(alkyl)aminocarbonyle ;
b) soit **Z₂** représente un groupement N⁺R⁵, **Z₁** représente un atome de carbone, **Z₃** et **Z₄** représentent un groupement CR⁴, avec R⁴ étant plus particulièrement un atome d'hydrogène ;
c) soit **Z₄** représente un groupement N⁺R⁵, **Z₁** représente un atome de carbone, **Z₃** et **Z₂** représentent un groupement CR⁴, avec R⁴ étant plus particulièrement un atome d'hydrogène ;
   et
d) soit **Z₁** représente un atome d'azote quaternisé N⁺, **Z₂** et **Z₄** représentent un groupement CR⁴ et **Z₃** représente un atome d'azote.

Un mode de réalisation particulier de l'invention concerne des colorants dérivés d'indole styryle à linker alkyle ou alkylène de formule **(Ia)** suivante : 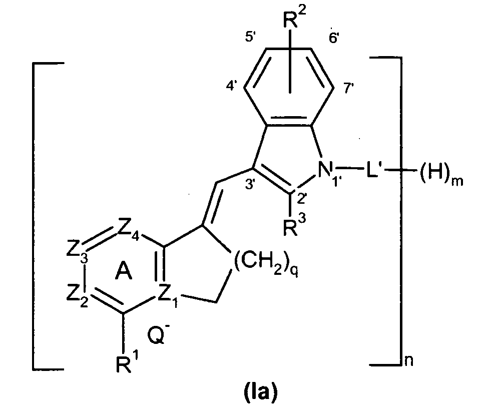 formule **(Ia)** dans laquelle :
➢ **n, m,** et **Q**⁻, sont tels que définis précédemment ;
➢ motif A dans lequel :
   a) soit **Z₁** représente un atome d'azote quaternisé N⁺, et **Z₂**, **Z₃** et **Z₄** représentent CR⁴, avec R⁴ étant plus particulièrement un atome d'hydrogène ou un groupement (C₁-C₄)alkyle ou **Z₃** et **Z₄** représentent CH et **Z₂** représente un groupement (C₁-C₆)alkylcarbonylamino ou (di)(C₁-C₄)(alkyl)aminocarbonyle ;
   b) soit **Z₂** représente un groupement N⁺R⁵, **Z₁** représente un atome de carbone, **Z₃** et **Z₄** représentent un groupement CR⁴, avec R⁴ étant plus particulièrement un atome d'hydrogène ; et
   c) soit **Z₄** représente un groupement N⁺R⁵, **Z₁** représente un atome de carbone, **Z₃** et **Z₂** représentent un groupement CR⁴, avec R⁴ étant plus particulièrement un atome d'hydrogène ; et
   c) soit **Z₁** représente un atome d'azote quaternisé N⁺, **Z₂** et **Z₄** représentent un groupement CR⁴ et **Z₃** représente un atome d'azote.
➢ **q** vaut 1 ou 2, particulièrement 1 ;
➢ **R¹**, représente un atome d'hydrogène, ou un groupement (C₁-C₆)alkyle particulièrement R¹ représente un atome d'hydrogène,
➢ **R²** est absent, ou représente un atome d'halogène tel que le chlore, un groupement (C₁-C₃)alkoxy, (C₁-C₃)alkoxycarbonyle ;particulièrement en position 5' ou 6'
➢ **R³**, représente un atome d'hydrogène, un groupement (C₁-C₆)alkyle tel que méthyle, un groupement phényle, un groupement (di)(C₁-C₆)(alkyl)amino tel que diméthylamino, ou un groupement hétérocycloalkyle à 5-7 chaînons, monocyclique comprenant de 1 à 3 hétéroatomes choisi parmi l'oxygène et l'azote éventuellement substitué par un groupement (C₁-C₂)alkyle tel que pipérazino ;
➢ **R⁵** représente un groupement (C₁-C₆)alkyle éventuellement substitué, ou un aryl(C₁-C₆)alkyle ; particulièrement R⁵ représente un groupement (C₁-C₄)alkyle tel que le méthyle ou l'éthyle ;
➢ **L'** représente une liaison σ, un chaîne (C₂-C₆)alkylène telle que éthylène -(CH₂)₂-, propylène -(CH₂)₃-, ou butylène -(CH₂)₄-, particulièrement éthylène ; un groupement (C₁-C₆)alkyle éventuellement substitué par notamment un groupement (di)(C₁-C₆)(alkyl)aminocarbonyle ;
étant entendu que lorsque n vaut 2 alors m vaut zéro, et lorsque n vaut 1 alors m vaut 1.

A titre d'exemple on peut citer les colorants fluorescents de formule (I) ou (la) suivants

| | |
|---|---|
| 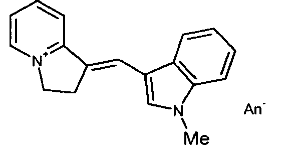 | **1** |
| 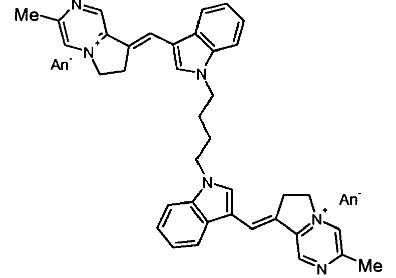 | **2** |
| | |
| 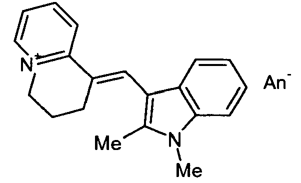 | **3** |
| 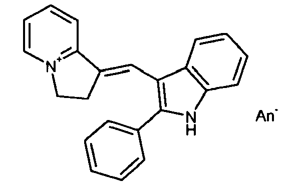 | **4** |
| 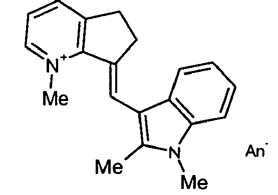 | **5** |
| 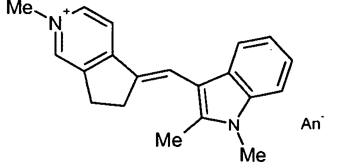 | **6** |
| 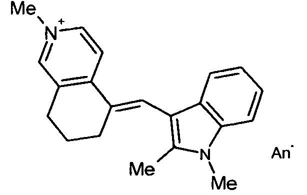 | **7** |
| 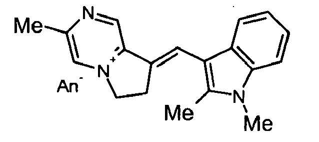 | **8** |
| 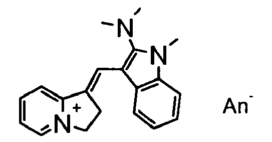 | **9** |
| 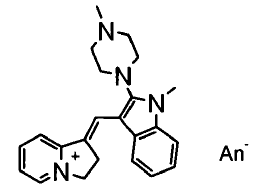 | **10** |
| 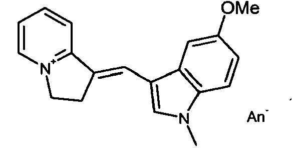 | **11** |
| 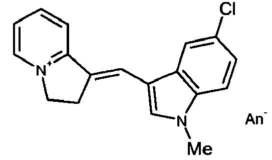 | **12** |
| 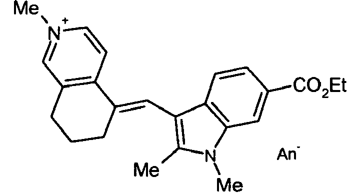 | **13** |
| 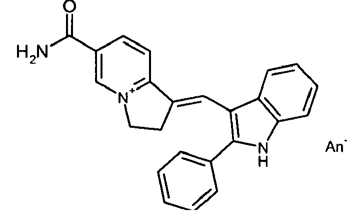 | **14** |
| 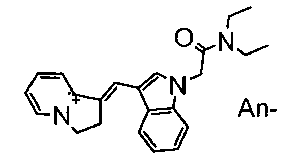 | **15** |
| 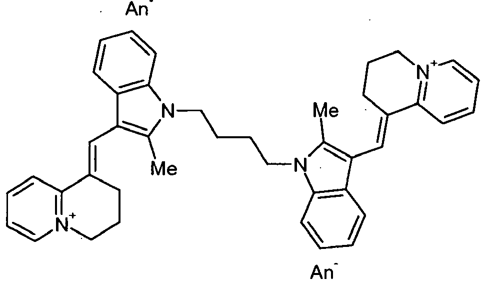 | **16** |
| 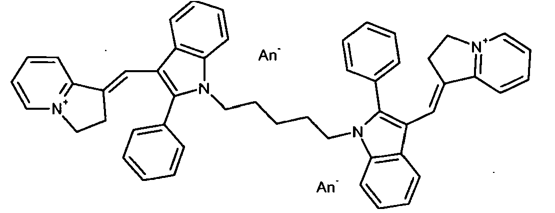 | **17** |
| 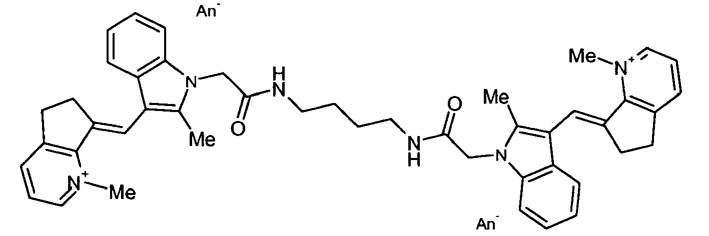 | **18** |
| 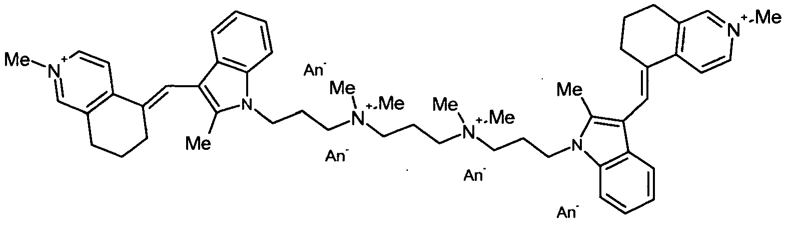 | **19** |
| 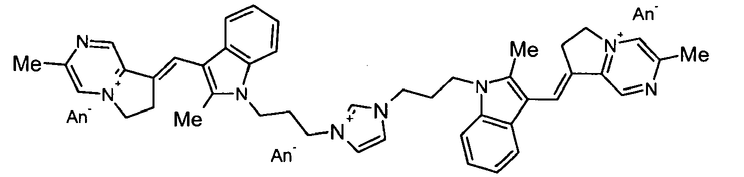 | **20** |
| 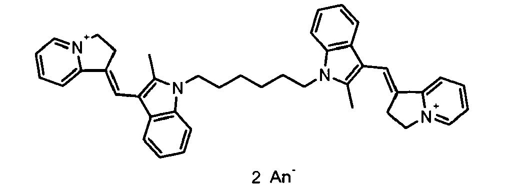 | **21** |

Avec An-, représentant un contre-ion anionique.

Pour tous les exemples des modes de réalisation de préparation qui suivent des nouveaux colorants dérivés d'indole styryle à linker alkyle ou alkylène de formule **(I)**, l'homme du métier sait protéger au préalable les fonctions réactives puis les déprotéger pour les besoins de la réaction de synthèse, par les méthodes classiques connues de protection/déprotection comme celles décrites dans les ouvrages cités ci-dessus de T.W Greene John Willey & Sons ed., NY, 1981, ou P. Kocienski « Protecting Groups », P. Kocienski, Thieme, 3ème ed., 2005.

Les colorants fluorescents de formule **(I)** peuvent être synthétisés en une étape par réaction d'un composé indole 3-carboxaldéhyde (**a**) avec un composé héteroaromatique cationique (**b**) pour conduire, par condensation de Knoevenagel, aux colorants styryles fluorescents **(I)**. 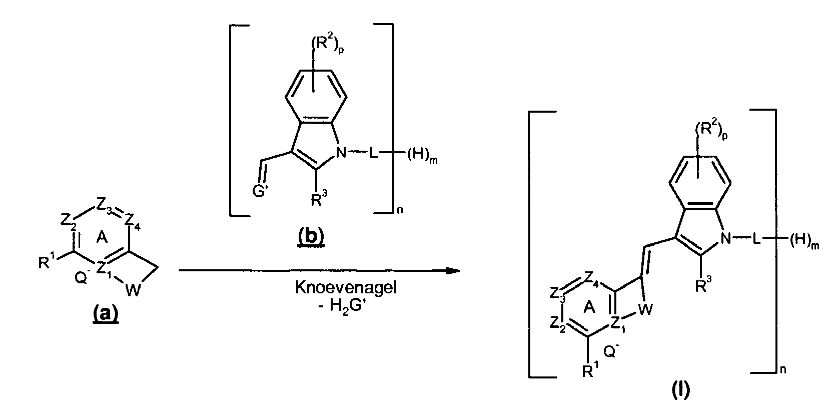 avec Z¹ à Z⁴, R¹ à R³ W, L, Q⁻, m, n et p sont tels que définis précédemment ; G' représentant un atome d'oxygène, de soufre ou un groupement NR' avec R' représentant un atome d'hydrogène ou un radical alkyle, et R représentant un atome d'hydrogène, un radical alkyle en C₁-C₄, hydroxyalkyle en C₁-C₄ ou un aryl(C₁-C₄)alkyle

Nous pouvons illustrer cette méthode par la synthèse des colorants fluorescents de formule **(I')** à partir de 2 équivalents de composé heteroaromatique cationique **(a)** et un équivalent de composé indole (**b)** comprenant deux fonctions électrophiles tel que aldéhyde, thioaldéhyde, pour conduire par double condensation de Knoevenagel aux colorants fluorescents **(Ia)**. 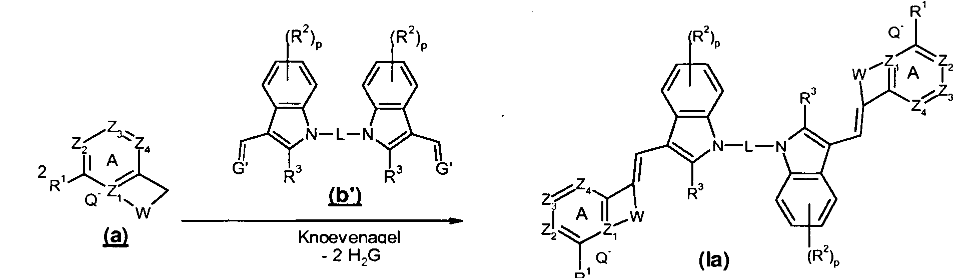 avec Z¹ à Z⁴, R¹ à R³, W, L, Q⁻, G' et p sont tels que définis précédemment.

Nous pouvons également illustrer par la synthèse des colorants fluorescents de formule **(Ib)** à partir d'un équivalent de composé hétéroaromatique cationique **(a)** et un équivalent de composé indole **(b")** comprenant un fonction électrophile tel que aldéhyde, thioaldéhyde, pour conduire par condensation de Knoevenagel aux colorants fluorescents **(Ib).** 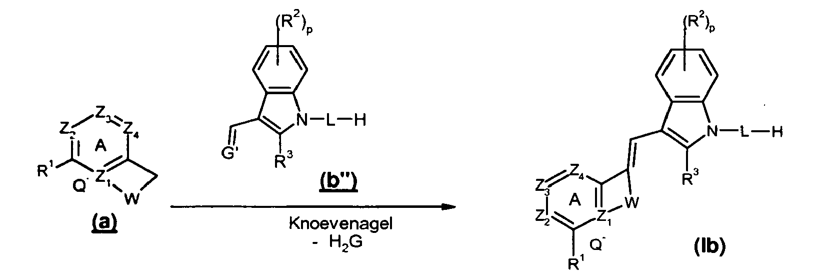 avec Z¹ à Z⁴, R¹ à R³, W, L, Q⁻, G' et p sont tels que définis précédemment.

On pourra se référer à l'ouvrage Advanced Organic Chemistry, « Reactions, Mechanisms and Structures », J. March, 4ème Ed, John Willey & Sons, 1992 pour avoir plus de détails sur les conditions opératoires mises en oeuvre pour le procédé mentionné ci-dessus.

Les réactifs de départs sont commerciaux ou accessibles par les méthodes classiques connues par l'homme du métier.

A titre d'exemple des composés héteroaromatiques commerciaux on peut citer le 5,6,7,8-TETRAHYDROISOQUINOLINE (CAS No. 36556-06-6), 5,6,7,8-TETRAHYDROQUINOLINE (CAS No.10500-57-9) et le 6,7-DIHYDRO-5H-CYCLOPENTA[B]PYRIDINE (CAS No. 533-37-9).

Ces composés héteroaromatiques commerciaux peuvent être quaternisés par les méthodes classiques connues par l'homme du métier pour former les composés héteroaromatiques cationiques (**b**).
A titre de exemple pour les voies de synthèse des composés héteroaromatiques cationiques (**b**) on peut citer J.O.C., 68(26),10123-10129, 2003 et Tetrahedron Letters, 31 (24), 3495-6, 1990 pour la synthèse des composés 2,3-dihydro-1H-indolizinium ; J.O.C. 70(21), 8508-12, 2005 ; Heterocycles 66,161-166, 2005 pour la synthèse des composés 1,2,3,4-tetrahydroquinolizinium ; Tetrahedron 46(7), 2561-72,1990 et J.O.C. 51(6), 872-5, 1986 pour la synthèse des composés 5,6,7,8-tetrahydroquinolinium et Faming Zhuanli Shenquing Gongkai Shuomingshu 1907973, 07 feb 2007 et J.O.C. 68(18) 6959-6966, 2003 pour la synthèse des composés 6,7-dihydro-5H-cyclopenta[b]pyridinium

A titre d'exemple des composés indole 3-carboxaldéhyde (**b**) commerciaux on peut citer I' INDOLE-3-CARBOXALDEHYDE (CAS No. 487-89-8), le 5-METHOXYINDOLE-3-CARBOXALDEHYDE (CAS No. 10601-19-1), le 2-METHYLINDOLE-3-CARBOXALDEHYDE (CAS No. 5416-80-8), le 1-METHYLINDOLE-3-CARBOXALDEHYDE (CAS No. 19012-03-4), le 1-METHYL-2-PHENYL-3-FORMYL INDOLE (CAS No. 1757-72-8) et le METHYL 3-FORMYLINDOLE-6-CARBOXYLATE (CAS No. 133831-28-4).

A titre d'exemple des composés indole 3-carboxaldéhyde (**b'**) commerciaux on peut citer l' 1,1-(2-hydroxy-1,3-propyldiyl)bis(1-H-indole-3-carboxaldehyde) (CAS No. 331275-74-2) et 1-(1,4-butandiyl)bis (1-H-indole-3-carboxaldehyde) (CAS No. 676244-31-8)

A titre de exemple pour les voies de synthèse des composés indole 3-carboxaldéhyde (b') on peut citer *Letters in* Organic Chemistry, 3(9), 712-704, 2006 et Organic & Biomolecular Chemistry, 2(19), 2874-2883, 2004
Selon une autre procédé de synthèse, on peut faire réagir un composé (e) comprenant une fonction nucléophile avec une quantité suffisante, préférentiellement équimolaire, d'un colorant fluorescent **(Ic)** qui comprend une fonction électrophile pour former une liaison covalente Σ; voir ci-dessous la préparation de colorants de formule **(Id)**: 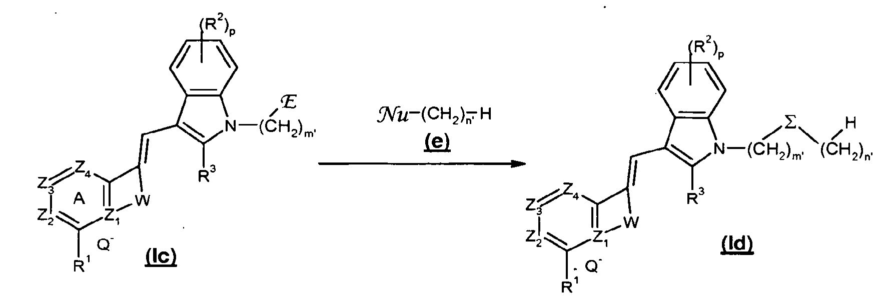 avec Z¹ à Z⁴, R¹ à R³, W, Q⁻ et p sont tels que définis précédemment ; m' et n' sont des entiers compris inclusivement entre 1 et 6 avec m'+n' vaut un entier compris entre 2 et 6, *Nu* représentant un groupement nucléophile ; *E* représentant un groupement électrophile ; et Σ représentant la liaison générée après attaque du nucléophile sur l'électrophile.

A titre d'exemple, les liaisons covalentes Σ pouvant être générées sont répertoriées dans le tableau ci-dessous à partir de condensation d'électrophiles avec des nucléophiles :

| Electrophiles *E* | Nucléophiles *Nu* | Liaisons covalentes Σ |
|---|---|---|
| Esters activés* | Amines | Carboxamides |
| Azotures d'acyles** | Amines | Carboxamides |
| Haloqénures d'acyles | Amines | Carboxamides |
| Haloqénures d'acyles | Alcools | Esters |
| Cyanures d'acyles | Alcools | Esters |
| Cyanures d'acyles | Amines | Carboxamides |
| Halogénures d'alkyles | Amines | Alkylamines |
| Halogénures d'alkyles | Acides carboxyliques | Esters |
| Halogénures d'alkyles | Thiols | Thioesters |
| Halogénures d'alkyles | Alcools | Ethers |
| Acides sulfoniques et leurs sels | Thiols | Thioéthers |
| Acides sulfoniques et leurs sels | Acides carboxyliques | Esters |
| Acides sulfoniques et leurs sels | Alcools | Ethers |
| Anhydrides | Alcools | Esters |
| Anhydrides | Amines | Carboxamides |
| Halogénures d'aryles | Thiols | Thioéthers |
| Halogénures d'aryles | Amines | Arylamines |
| Aziridines | Thiols | Thioéthers |
| Acides carboxyliques | Amines | Carboxamides |
| Acides carboxyliques | Alcools | Esters |
| Carbodiimides | Acides carboxyliques | N-acylurées |
| Diazoalcanes | Acides carboxyliques | Esters |
| Epoxides | Thiols | Thioéthers |
| Haloacétamides | Thiols | Thioéthers |
| Esters imidiques | Amines | Amidines |
| Isocyanates | Amines | Urées |
| Isocyanates | Alcools | Uréthanes |
| Isothiocyanates | Amines | Thiourées |
| Maléimides | Thiols | Thioéthers |
| Esters sulfoniques | Amines | Alkylamines |
| Esters sulfoniques | Thiols | Thioéthers |
| Esters sulfoniques | Acides carboxyliques | Esters |
| Esters sulfoniques | Alcools | Ethers |
| Halogénures de sulfonyle | Amines | Sulfonamides |

| | | |
|---|---|---|
| **les esters activées de formule générale -CO-Part avec Part représentant un groupement partant tel que oxysuccinimidyle, oxybenzotriazolyle, aryloxy éventuellement substitué ;* *** les azotures d'acyles peuvent* se *réarranger pour donner les isocyanates.* | | |

Une variante à ce procédé est d'utiliser un colorant styryle possédant une fonction acrylate électrophile (-OCO-C=C-) sur laquelle on effectue une réaction d'addition qui génèrera une liaison Σ.

Une variante à ce procédé est d'utiliser un colorant styryle (le) possédant une fonction nucléophile sur laquelle on réagit une composé (f) qui comprend une fonction électrophile pour former une liaison covalente Σ; voir ci-dessous la préparation de colorants de formule (If): 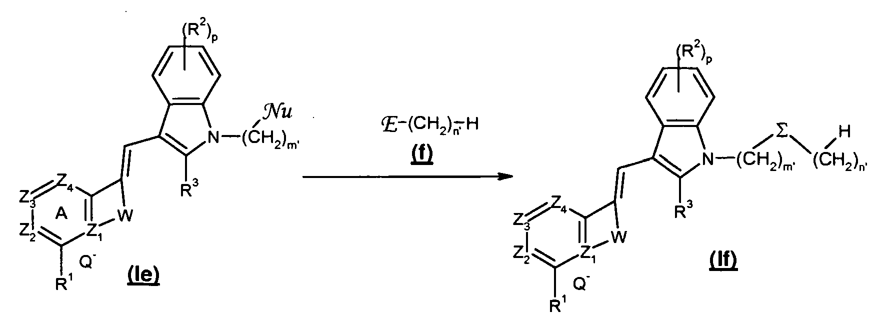 avec Z¹ à Z⁴, R¹ à R³, W, Q⁻, L, *Nu* ; *E*, Σ, p, sont tels que définis précédemment.

Une variante à ce procédé est de faire réagir deux equivalents d'un colorant styryle (le) possédant une fonction nucléophile avec une équivalente d' un composé (g) qui comprend deux fonctions électrophiles pour former deux liaisons covalentes Σ; voir ci-dessous la préparation de colorants de formule (Ig):

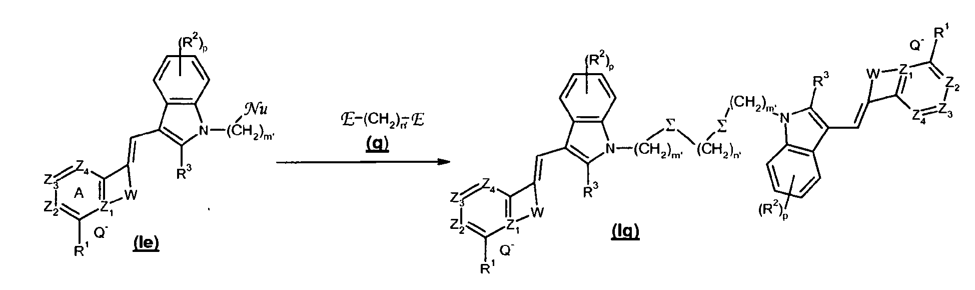

Avec Z¹ à Z⁴, R¹ à R³, W, Q⁻, *Nu*; *E*, Σ, p, n' et m', tels que définis précédemment,

Une autre variante est de faire réagir deux équivalents d'un colorant styryle (Ic) possédant une fonction électrophile avec une équivalente d'un composé (h) qui comprend deux fonctions nucléophiles pour former deux liaisons covalentes Σ; voir ci-dessous la préparation de colorants de formule (Ih):

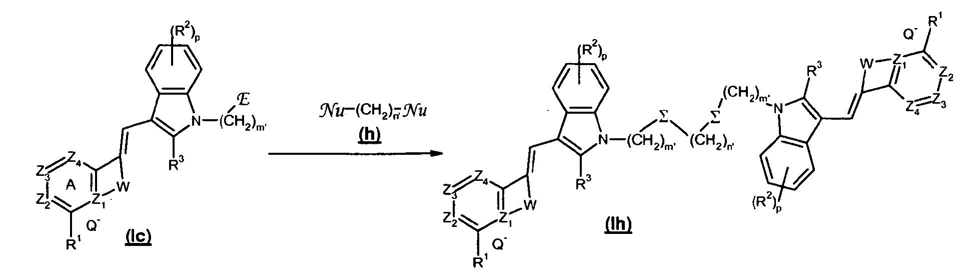

Avec Z¹ à Z⁴, R¹ à R³, W, Q⁻, *Nu*; *E*, Σ, p, n' et m', tels que définis précédemment,

Une autre variante est de faire réagir un colorant styryle (Ic) possédant une fonction électrophile avec un colorant styryle (le) possédant une fonction nucléophile pour former un colorant (li) avec un liaison covalente Σ; voir ci-dessous la préparation de colorants de formule (li): 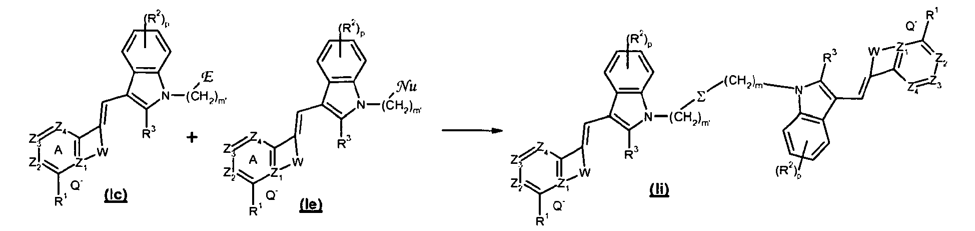 Avec Z¹ à Z⁴, R¹ à R³, W, Q⁻, *Nu*; *E*, Σ, p, n' et m', tels que définis précédemment,

Conformément à une autre possibilité, les colorants fluorescents **(Ij))** peuvent être obtenus par réaction d'un composé **(i)** comprenant un groupement partant *Gp* nucléofuge, comme par exemple mésylate, tosylate, triflate ou halogénure avec un chromophore **(Ik)**.

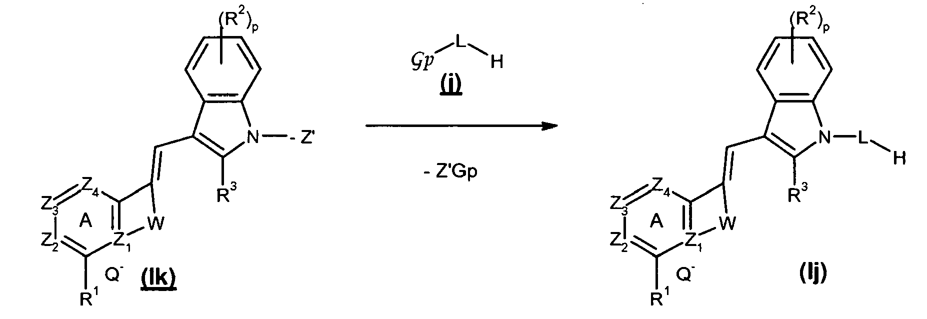

Avec Z¹ à Z⁴, R¹ à R³, W, Q⁻, L et p sont tels que définis précédemment, et Z' représente un atome d'hydrogène ou un groupement activant la nucléophilie de X.

On pourra se référer à l'ouvrage Advanced Organic Chemistry, « Reactions, Mechanisms and Structures », J. March, 4ème Ed, John Willey & Sons, 1992 ou T. W. Greene « Protective Groups in Organic Synthesis », pour avoir plus de détails sur les conditions opératoires mises en oeuvre pour les procédés mentionnés ci-dessus.

Un autre objet de l'invention concerne une composition qui contient au moins un colorant dérivé d'indole styryle à linker alkyle ou alkylène de formule **(I)**.
Plus particulièrement, la composition tinctoriale utile dans l'invention contient en général une quantité de colorant fluorescent de formule **(I)** comprise entre 0,001 et 50% par rapport au poids total de la composition. De préférence, cette quantité est comprise entre 0,005 et 20% en poids et encore plus préférentiellement entre 0,01 et 5% en poids par rapport au poids total de la composition.
La composition tinctoriale peut en outre contenir des colorants directs additionnels. Ces colorants directs sont par exemple choisis parmi les colorants directs nitrés benzéniques neutres, acides ou cationiques, les colorants directs azoïques neutres, acides ou cationiques, les colorants tétraazapentaméthiniques, les colorants quinoniques et en particulier anthraquinoniques neutres, acides ou cationiques, les colorants directs aziniques, les colorants directs triarylméthaniques, les colorants directs indoaminiques et les colorants directs naturels.
Parmi les colorants directs naturels, on peut citer la lawsone, la juglone, l'alizarine, la purpurine, l'acide carminique, l'acide kermésique, la purpurogalline, le protocatéchaldéhyde, l'indigo, l'isatine, la curcumine, la spinulosine, l'apigénidine. On peut également utiliser les extraits ou décoctions contenant ces colorants naturels et notamment les cataplasmes ou extraits à base de henné.
La composition tinctoriale peut contenir une ou plusieurs bases d'oxydation et/ou un ou plusieurs coupleurs conventionnellement utilisés pour la teinture de fibres kératiniques.
Parmi les bases d'oxydation, on peut citer les para-phénylènediamines, les bis-phénylalkylènediamines, les para-aminophénols, les bis-para-aminophénols, les ortho-aminophénols, les bases hétérocycliques et leurs sels d'addition
Parmi ces coupleurs, on peut notamment citer les méta-phénylènediamines, les méta-aminophénols, les méta-diphénols, les coupleurs naphtaléniques, les coupleurs hétérocycliques et leur sels d'addition.
Le ou les coupleurs sont chacun généralement présents en quantité comprise entre 0,001 et 10% en poids du poids total de la composition tinctoriale, de préférence entre 0,005 et 6%.
La ou les bases d'oxydation présentes dans la composition tinctoriale sont en général présentes chacune en quantité comprise entre 0,001 à 10% en poids du poids total de la composition tinctoriale, de préférence entre 0,005 et 6% en poids.
D'une manière générale, les sels d'addition des bases d'oxydation et des coupleurs utilisables dans le cadre de l'invention sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates et les sels d'addition avec une base telles que les hydroxydes de métal alcalin comme la soude, la potasse, l'ammoniaque, les amines ou les alcanolamines.
Le milieu cosmétique approprié pour la teinture, appelé aussi support de teinture, est un milieu cosmétique généralement constitué par de l'eau ou par un mélange d'eau et d'au moins un solvant organique. A titre de solvant organique, on peut par exemple citer les alcanols inférieurs en C₁-C₄, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.
Les solvants lorsqu'ils sont présents sont, de préférence présents dans des proportions comprises entre 1 et 40% en poids environ par rapport au poids total de la composition tinctoriale, et encore plus particulièrement entre 5 et 30% en poids environ.
La composition tinctoriale peut également renfermer divers adjuvants utilisés classiquement dans les compositions pour la teinture des cheveux, tels que des agents tensioactifs anioniques, cationiques, non ioniques, amphotères, zwittérioniques ou leurs mélanges, des polymères anioniques, cationiques, non ioniques, amphotères, zwittérioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non tels que les silicones aminés, des agents filmogènes, des céramides, des agents conservateurs, des agents opacifiants, des polymères conducteurs.
Les adjuvants ci dessus sont en général présents en quantité comprise pour chacun d'eux entre 0,01 et 20% en poids par rapport au poids de la composition.
Bien entendu, l'homme de l'art veillera à choisir ce ou ces éventuels composés complémentaires de manière telle que les propriétés avantageuses attachées intrinsèquement à la composition de teinture conforme à l'invention ne soient pas, ou substantiellement pas, altérées par la ou les adjonctions envisagées.
Le pH de la composition tinctoriale est généralement compris entre 3 et 14 environ, et de préférence entre 5 et 11 environ. Il peut être ajusté à la valeur désirée au moyen d'agents acidifiants ou alcalinisants habituellement utilisés en teinture des fibres kératiniques ou bien encore à l'aide de systèmes tampons classiques.
Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.
Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (y) suivante : 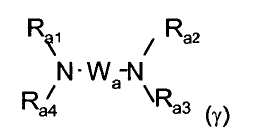 dans laquelle Wₐ est un reste propylène éventuellement substitué par un groupement hydroxy ou un radical alkyle en C₁-C₄ ; Rₐ₁, Rₐ₂, Rₐ₃ et Rₐ₄, identiques ou différents, représentent un atome d'hydrogène, un radical alkyle en C₁-C₄ ou hydroxyalkyle en C₁-C₄.
La composition tinctoriale peut se présenter sous des formes diverses, telles que sous forme de liquide, de crème, de gel, ou sous toute autre forme appropriée pour réaliser une teinture des fibres kératiniques, et notamment des cheveux.

Un autre objet de l'invention est un procédé de coloration avec effet éclaircissant de fibres kératiniques notamment foncées consistant à appliquer sur lesdites fibres une composition tinctoriale comprenant au moins un colorant fluorescent de formule **(I)** ou (la) tels que définis précédemment.
L'application de la composition tinctoriale selon l'invention est généralement effectuée à température ambiante. Elle peut cependant être réalisée à des températures variant de 20 à 180°C.
Les exemples qui suivent servent à illustrer l'invention sans toutefois présenter un caractère limitatif. Les colorants des exemples ci-après ont été entièrement caractérisés par les méthodes spectroscopiques et spectrométriques classiques.

### EXEMPLES

### Exemple 1 : Chlorure de 1-[(1-méthyl-1H-indol-3-yl)méthylène]-2,3-dihydro-1H-indolizinium [1]

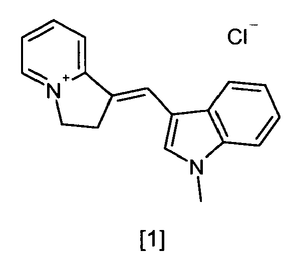

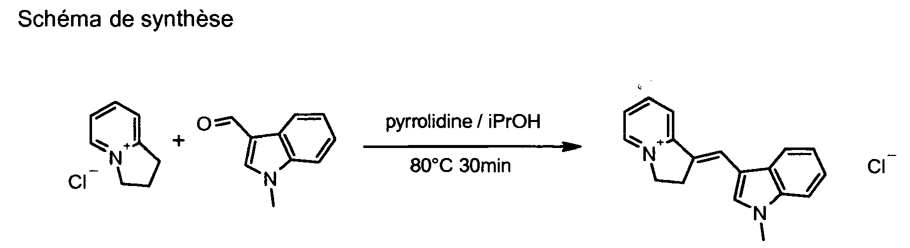

Dans un tricol de 50mL, 1.55g de chlorure de 2,3-dihydro-1H-indolizinium et 1.59 g de 1-méthylindole-3-carboxaldéhyde sont mis en suspension dans 15 mL d'isopropanol. Le mélange réactionnel est chauffé à 80 °C puis 820 µL de pyrrolidine sont introduits. Le mélange réactionnel est refroidi immédiatement à température ambiante. Le précipité est filtré, lavé avec 2 fois 30mL d'isopropanol avant d'être séché sous vide poussé sous P₂O₅ jusqu'à masse constante. On recueille 2.59 g d'une poudre orange. Les analyses (RMN (DMSO d6)) et Spectrometrie de Masse ESI+ M/Z=261) conforment à la structure attendue.

### Exemple 2 : Acétate de 1-{[1-méthyl-2-(4-méthylpipérazin-1-yl)-1H-indol-3-yl]méthylène}-2,3-dihydro-1 H-indolizinium [2]

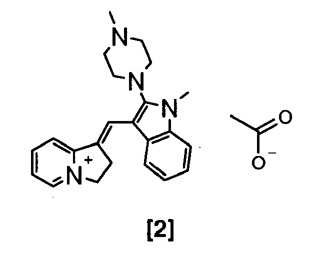

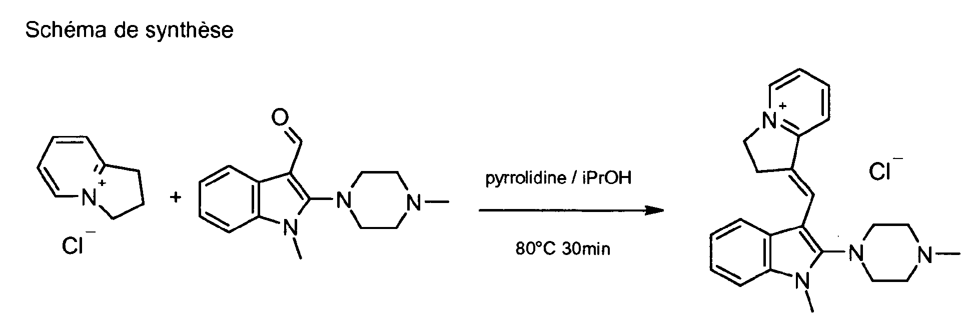

0.395g de 1-méthyl-2-(4-méthylpipérazino)-1H-indole-3-carbaldéhyde est mis en solution dans 7 mL d'isopropanol. 0.138 g de pipéridine est ajouté puis 0.218g de chlorure de 2,3-dihydro-1H-indolizinium dissout dans 7 mL d'isopropanol. Les mélanges réactionnels sont portés sous agitation à 80°C pendant 24H. 50 ml d'acétate d'éthyle est ajouté afin de précipiter le colorant. Le précipité est filtré, lavé 3 fois avec 25 ml d'acétate d'éthyle avant d'être séché sous vide jusqu'à masse constante. La poudre orange est purifiée par chromatographie. Les analyses conforment à la structure attendue.
Spectrometrie de Masse : Le cation attendu [C23H27N4]+ est principalement détecté. ESP+=180, 201 et 359 (correspondant à [M+H]2+, [M+H+CH3CN]2+, et [M]+),
RMN : Le spectre est en accord avec la structure attendue.
HPLC : une pureté UV relative supérieure à 95%. Lamda max=408 nm.

### Exemple 3 : chlorure de 1-({1-[2-(diéthy)amino)-2-oxoéthy)]-1H-indol-3-yl}méthylène)-2,3-dihydro-1H-indolizinium chloride [3]

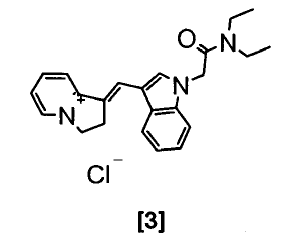

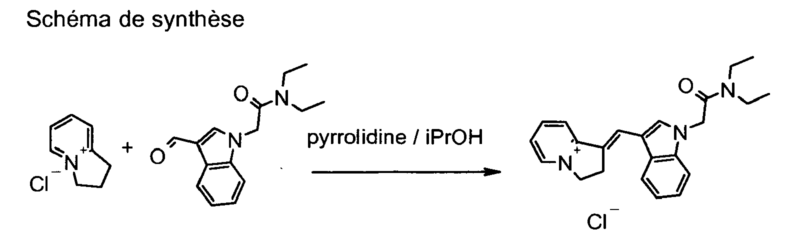

0.800g de N,N-diethyl-2-(3-formyl-1 H-indol-1-yl) acétamide est mis en solution dans 4,2 mL d'isopropanol. 0.238 g de pipéridine est ajouté puis 0.435g de chlorure de 2,3-dihydro-1H-indolizinium dissout dans 7 mL d'isopropanol. Les mélanges réactionnels sont portés sous agitation à 80°C pendant 24H. 50 ml d'acétate d'éthyle est ajouté afin de précipiter le colorant. Le précipité est filtré, lavé 3 fois avec 25 ml d'acétate d'éthyle avant d'être séché sous vide jusqu'à masse constante. On recueille 1,09g de poudre orange. Les analyses sont conformes à la structure attendue.

### Exemple 4 - mésylate de 5-[(1,2-diméthyl-1H-indol-3-yl)méthylidènel-2-méthyl-5,6,7,8-tétrahydroisoqunolinium

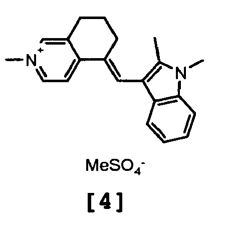

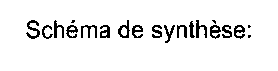

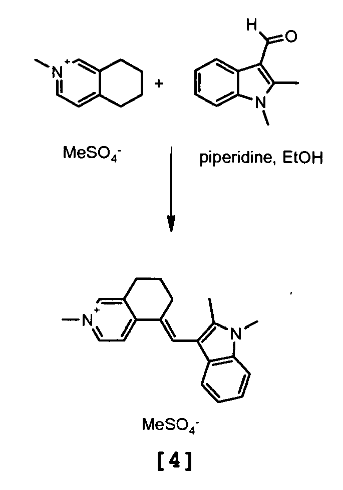

A une solution sous agitation comprenant le mésylate de 2-méthyl-5,6,7,8-tétrahydroisoquinolinium (0.273 g, 1 mmol) et 1,2-diméthyl-1*H-*indole-3-carbaldéhyde (0.260 g, 1.5 mmol) dans 5 ml d'éthanol est ajouté 0.1 ml de pipéridine comme catalyseur. Le mélange réactionnel est chauffé à reflux pendant 20 h. Après refroidissment, les solvants sont évaporés sous vide. Le résidu est alors chromatographié sur alumine (éluant: dichlorométhane / méthanol = 100/1 à 50/1) deux fois pour conduire à 0.1 g de composé **[4]** pur. Les analyses sont conformes à la structure attendue.

### Exemple 5-dibromure de (1,1'-{hexane-1,6-diylbis[(2-méthyl-1H-indole-1,3-diyl)méthylylidène]}bis(2,3-dihydro-1H-indolizinium) [5]

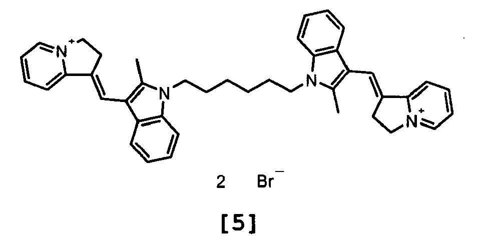

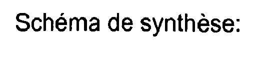

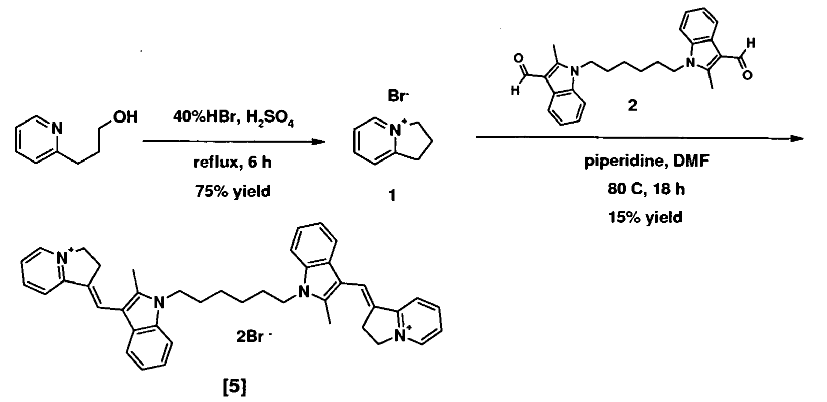

*Synthèse du composé **1:*** Un mélange de 4.11g (30 mmol) de 2-(3-hydroxypropyl)pyridine, 13.7 ml (120 mmol) de 48% d'acide hydrobromique (HBr), et 3.3 ml (60 mmol) d'acide sulfurique concentré sont chauffés à reflux pendant 6 h. L'eau est retirée et de l'éthanol est ajouté. Le brut est ensuite filtré et le filtrat concentré sous vide. Le résidu ést chromatographié sur silica gel (éluant: CH₂Cl₂ / MeOH = 10/1) pour obtenir 4.5 g de produit désiré 1 avec un rendement de 75%.

*Synthèse de composé 2:* A une solution sous agitation de 2-méthylindole-3-carboxaldéhyde (6.36 g, 40 mmol) dans 90 ml de THF est ajouté petit à petit 2.7 g (67.5 mmol) d'hydrure de sodium (NaH). Après agitation d'une demi-heure, 2.3 ml (15 mmol) de 1,6-dibromohexane sont ajoutés. Le mélange réactionnel est laissé sous agitation pendant 22 h à température ambiante. La réaction est ensuite stoppée, neutralisée par addition d'un peu d'eau. La phase organique est extraite au dichlorométhane trois fois, séchée sur sulfate de magnesium anhydre (MgSO₄), filtrée, puis le solvant est évaporé. Le résidu est purifié par chromatographie sur silice (eluant: CH₂Cl₂ / MeOH = 10/1) un brut qui après recristallisation dans l'éthanol conduit à 2.8 g de composé 2 avec un rendement de 63%.

*Synthèse du composé **[5]**:* A une solution sous agitation comprenant les composés précédemment synthétisés 1 (0.4 g, 2 mmol) et 2 (0.35 g, 0.88 mmol) dans 10 ml de DMF il est ajouté 0.5 ml de pipéridine comme catalyseur. Le mélange réactionnel est chauffé à 80 °C pendant 18 h. Après refroidissement du mélange, le DMF est évaporé sous vide. Le résidu est purifié par chromatographie sur alumine (éluant: CH₂Cl₂ / MeOH = 500/5 à 500/10) puis purifié à nouveau par recristallisation dans une mélange de solvants (EtOH / EtOAc = 1/3) pour conduire à 0.1 g du produit **[5]** attendu.
Les analyses sont en accord avec la structure du composé **[5].**

### EXEMPLE DE COLORATION

### Exemple 1 : Procédé de coloration - composé [1]

On a préparé la composition tinctoriale dans les proportions suivantes
Solution 1

| | |
|---|---|
| Hydroxyéthylcellulose Natrosol 250MR | 0.72g |
| Alkyl C8/C10(50 :50) hydroxyethylcellulose CG110 | 5g |
| Alcool benzylique | 4g |
| Polyethylène glycol 400 | 4g |
| Eau | Qsp 100g |

Solution 2 : TAMPON pH 7

| | |
|---|---|
| KH2PO4 | 0,026mol/l |
| Na2PO4 | 0,041mol/l |
| Eau déminéralisée | qsp 500ml |

La composition de coloration est obtenue en dissolvant le colorant [1] (5x10-3 mol/l) dans la solution 1 puis en ajoutant un volume équivalent de solution tampon 2.
La composition est appliquée sur des cheveux gris à 90% de blancs (BN), des cheveux décolorés et des cheveux foncés (hauteur de ton 4 HT4). Après 30 min de pause, les mèches sont rincées, lavées avec un shampooing standard, rincées à nouveau puis séchées.

### Observations visuelles

### Coloration :

La mèche de hauteur de ton 4 est devenue visuellement plus claire que des mèches témoins non traitées.
Les mèches de cheveux gris à 90% de blancs (BN) et de cheveux décolorés sont colorées avec des nuances intenses :

| composé [1] | Couleur obtenue |
|---|---|
| cheveux gris à 90% de blancs (BN) | jaune-orangé vif |
| cheveux décolorés | jaune-orangé vif |

Lors du rinçage et des shampooings des exemples [1], il y a très peu de dégorgement visible, la mousse des shampooings et les eaux de rinçage sont très peu colorées.
La couleur jaune-orangé est conservée sur les cheveux gris à 90% et les cheveux décolorés.

L'effet d'éclaircissement sur les cheveux foncés (hauteur de ton 4 HT4) reste visible.

## Revendications

1. Colorant fluorescent de formule (**I**) ou (**Ia**): 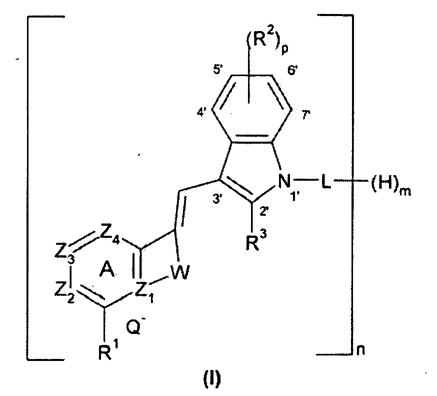 leurs sels d'acide organique ou minéral, isomères optiques, isomères géométriques, et les solvates tels que hydrates;
formule (**I**) dans laquelle:
➢ **n** représente 1 ou 2 :
➢ **m** représente 0 ou 1 ;
➢ **p** représente un entier compris inclusivement entre 0 et 4 ;
➢ **Q⁻** représente un contre-ion anionique ;
➢ **W** représente une chaîne (C₂-C₃)alkylène ou (C₂-C₃)alkénylène éventuellement substituée par un ou plusieurs groupement (C₁-C₄)alkyle ou aryle ;
➢ **Z₁** représente un atome de carbone ou d'azote quaternisé N⁺ ;
➢ **Z₂, Z₃, Z₄,** identiques ou différents, représentent un atome d'azote, un groupement CR⁴ ou N⁺R⁵ ; étant entendu qu'un seul atome d'azote quaternisé N⁺ ou N⁺R⁵ ne peut être présent dans le cycle aromatique A ;
➢ **R¹; R²** et **R⁴,** identiques ou différents, représentent un atome d'halogène, un groupement (di)(C₁-C₆)(alkyl)amino, cyano, hydroxy, (poly)halogéno(C₁-C₆)alkyle tel que trifluorométhyle, acyl(C₁-C₄)(alkyl)amino, (C₁-C₆)alcoxy, (C₁-C₆)alkylthio, (poly)hydroxy(C₂-C₆)alcoxy, (C₁-C₆)alkylcarbonyloxy, (C₁-C₆)alcoxycarbonyle, (C₁-C₆)alkylcarbonylamino_{;} (di)(C₁-C₄)(alkyl)aminocarbonyle, (C₁-C₆)alkylsuifonyl(C₁-C₄)(alkyl)amino, (di)(C₁-C₄)(alkyl)aminosulfonyle, ou un groupement (C₁-C₆)alkyle éventuellement substitué par un groupement choisi parmi (C₁C₆)alcoxy, hydroxy, (di)(C₁-C₆)(alkyl)amino ; ou alors lorsque p est une entier supérieur ou égal à 2, deux radicaux R² contigus forment ensemble avec l'atome de carbone qui les portent un cycle benzo ;
➢ **R³** représente un atome d'hydrogène, un groupement (C₁-C₆)alkyle éventuellement substitué, (di)(C₁-C₆)(alkyl)amino, aryle, hétérocycloalkyle éventuellement substitué ;
➢ **R⁵** représente un groupement (C₁-C₆)alkyle éventuellement substitué, ou un aryl(C₁-C₆)alkyle ;
➢ **L** représente un liaison σ ou une chaîne hydrocarbonée bivalente en C₁-C₂₀, éventuellement substituée, éventuellement interrompue i) par un ou plusieurs groupes divalents ou leurs combinaisons choisis parmi : -N(R)- ; -N⁺(R)(R°)-. An : -O-; -C(O)- et -S(O)₂- avec R, R°, identiques ou différents, choisi parmi un hydrogène, un radical (C₁-C₄)alkyle, hydroxy(C₁-C₄)alkyle, amino(C₁-C₄)alkyle et An représentant un contre-ion anionique ou ii) par un hétérocycle cationique ou hétéroaryle cationique Hét⁺, An⁻, avec An⁻ représentant un contre-ion anionique et Het⁺ représentant un hétérocycle comprenant de 5 à 10 chaînons, saturé ou non, ou un hétéroaryle comprenant de 5 à 10 chaînons ;
étant entendu que lorsque n vaut 2 alors m vaut zéro, et lorsque n vaut 1 alors m vaut 1; et 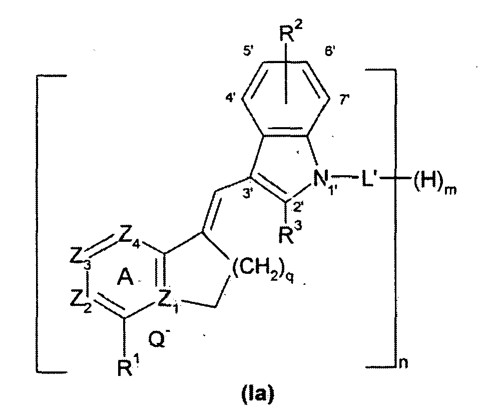 formule **(Ia)** dans laquelle :
➢ **n, m,** et **Q⁻,** sont tels que définis dans la revendication 1;
➢ motif A dans lequel:
a) soit **Z₁,** représente, un atome d'azote quaternisé N⁺, **et Z₂, Z₃** et Z₄ représentent CR⁴ , avec R⁴ étant un atome d'hydrogène ou un groupement (C₁-C₄) alkyle, ou **Z₃ et Z₄** représentent CH et **Z₂** représente un groupement (C₁-C₆)alkylcarbonylamino ou (di)(C₁-C₄)(alkyl)aminocarbonyle;
b) soit **Z₂** représente un groupement N⁺R⁵ **Z₁** représente un atome de carbone, **Z₃** et **Z₄** représentent un groupement CR⁴ avec R⁴ étant un atome d'hydrogène,
c) soit **Z₄** représente un groupement N⁺R⁵, **Z₁** représente un atome de carbone, **Z₃** et **Z₂** représentent un groupement CR⁴ avec R⁴ étant un atome d'hydrogène, et
d) soit, **Z₁** représente un atome d'azote quaternisé N⁺, **Z₂** et **Z₄** représentent un groupement CR⁴ et **Z₃** représente un atome d'azote.
➢ **q** vaut 1 ou 2, particulièrement 1 ;
➢ **R¹,** représente un atome d'hydrogène, ou un groupement (C₁-C₆)alkyle particulièrement R¹ représente un atome d'hydrogène,
➢ **R²** est absent, ou représente un atome d'halogène, un groupement (C₁-C₃)alkoxy, (C₁-C₃)alkoxycarbonyle ;
➢ **R³,** représente un atome d'hydrogène, un groupement (C₁-C₆)alkyle tel que méthyle, un groupement phényle, un groupement (di)(C₁-C₆)(alkyl)amino tel que diméthylamino, ou un groupement hétérocycloalkyle à 5-7 chaînons, monocyclique comprenant de 1 à 3 hétéroatomes choisi parmi l'oxygène et l'azote éventuellement substitué par un groupement (C₁-C₂)alkyle tel que pipérazino ;
➢ **R⁵** représente un groupement (C₁-C₆)alkyle événtuellement substitué, ou un aryl(C₁-C₆)alkyle; particulièrement R⁵ représente un groupement (C₁-C₄)alkyle tel que le méthyle ou l'éthyle ;
➢ **L'** représente une liaison σ, un chaîne (C₂-C₆)alkylène telle que éthylène - (CH₂)₂-; propylène -(CH₂)₃-, ou butylène -(CH₂)₄-, particulièrement éthylène ; un groupement (C₁-C₆)alkyle éventuellement substitué par notamment un groupement (di)(C₁-C₆)(alkyl)aminocarbonyle ;
étant entendu que lorsque n vaut 2 alors m vaut zéro, et lorsque n vaut 1 alors m vaut 1.

2. Colorant fluorescent de formule (**I**) selon la revendication précédente dans lequel m et n valent 1.

3. Colorant fluorescent de formule (1) selon la revendication 1 dans lequel n=2 et m=0.

4. Colorant fluorescent de formule (**I**) selon l'une quelconque des revendications précédentes comprenant un motif A dans lequel :
a) soit **Z₁** représente un atome d'azote quaternisé **N⁺,** et **Z₂, Z₃** et **Z₄** représentent CR⁴, ou **Z₃** et **Z₄** représentent CH et **Z₂** représente un groupement (C₁-C₆)alkylcarbonylamino ou (di)(C₁-C₄)(alkyl)aminocarbonyle
b) **soit Z₂** représente un groupement N⁺R⁵, **Z₁** représente un atome de carbone, **Z₃** et **Z₄** représentent un groupement CR⁴,
c) soit **Z₄** représente, un groupement N⁺R⁵, **Z₁** représente un atome de carbone, **Z₃** et **Z₂** représentent un groupement CR⁴, et
d) soit **Z₁** représente un atome d'azote quaternisé N⁺, **Z₂** et **Z₄** représentent un groupement CR⁴ et **Z₃** représente un atome d'azote.

5. Colorant fluorescent de formule (**I**) ou (**Ia**) selon l'une quelconque des revendications précédentes, choisi parmi les colorants suivants:
| | |
|---|---|
| 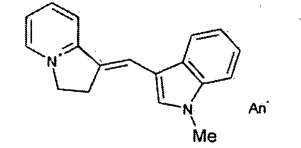 | **1** |
| 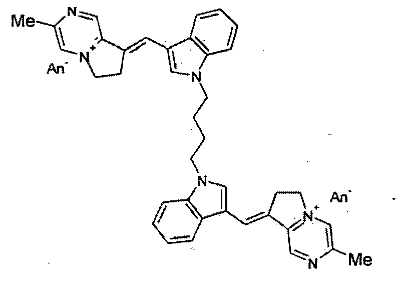 | **2** |
| | |
| 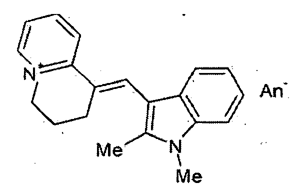 | **3** |
| 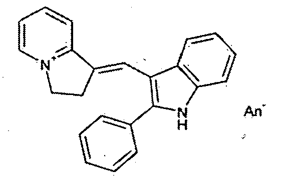 | **4** |
| 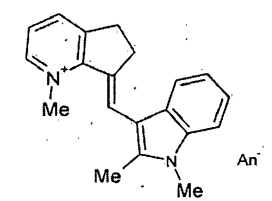 | **5** |
| 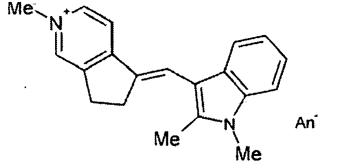 | **6** |
| 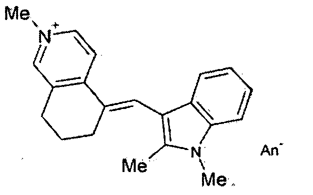 | **7** |
| 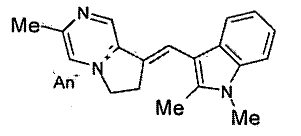 | **8** |
| 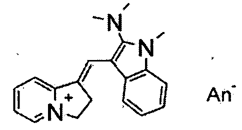 | **9** |
| 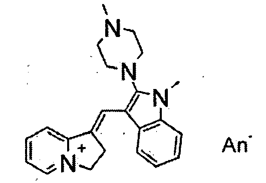 | **10** |
| 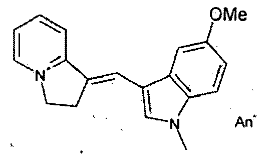 | **11** |
| 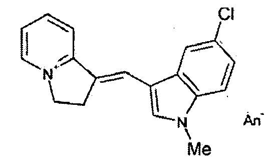 | **12** |
| 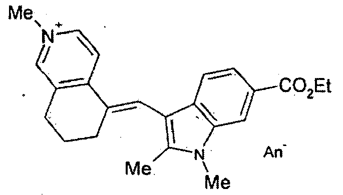 | **13** |
| 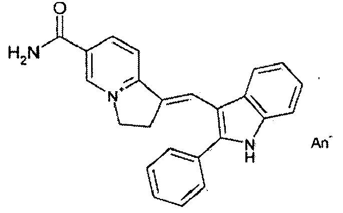 | **14** |
| 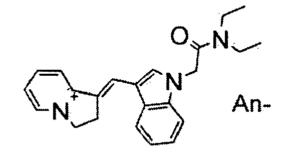 | **15** |
| 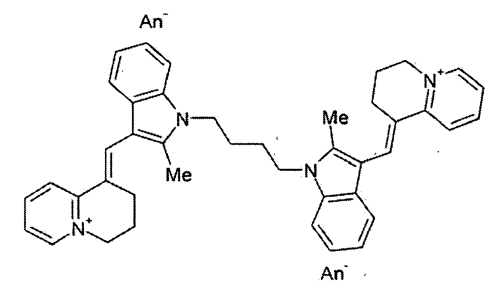 | **16** |
| 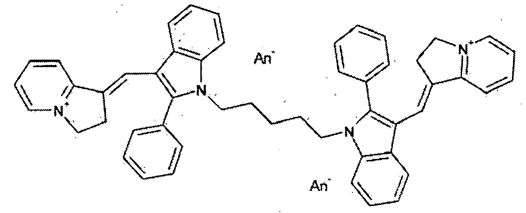 | **17** |
| 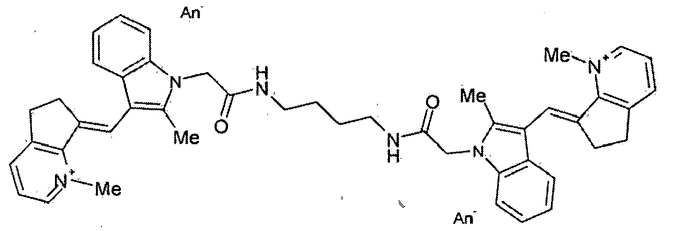 | **18** |
| 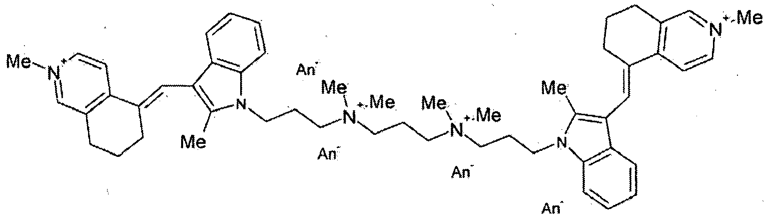 | **19** |
| 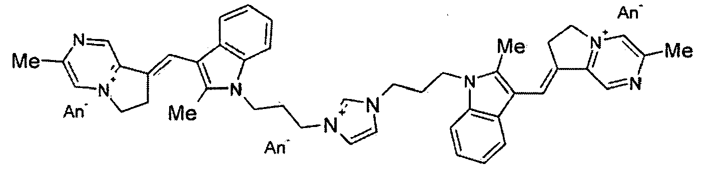 | **20** |
| 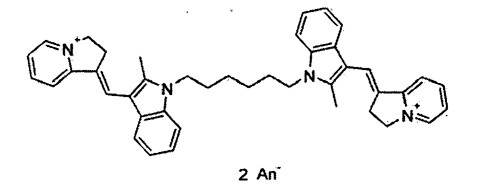 | **21** |
avec An⁻, identiques ou différents, représentant un contre-ion anionique.

6. Composition tinctoriale comprenant dans un milieu cosmétique approprié; un colorant fluorescent de formule **(I)** ou **(Ia)** tel que défini dans une quelconque des revendications 1 à 5.

7. Composition selon la revendication précédente, dans laquelle le colorant de formule (I) ou (Ia) est présent en quantité comprise entre 0,001 et 50% en poids par rapport au poids total de la composition.

8. Procédé de coloration de matières kératiniques, dans lequel on applique sur les matières une composition tinctoriale cosmétique comprenant au moins un colorant de formule **(I)** ou **(Ia)** selon une quelconque des revendications 6 ou 7.

9. Procédé de coloration de matières kératiniques selon la revendication précédente **caractérisé en ce que** les matières kératiniques sont des fibres kératiniques foncées possédant une hauteur de ton inférieure ou égale à 6.

10. Utilisation des colorants de formule **(I)** ou **(Ia)** tels que définis aux revendications 1 à 6 pour la teinture de fibres kératiniques humaines foncées.

11. Utilisation selon la revendication précédente pour l'éclaircissement des fibres kératiniques foncées.

12. Utilisation selon les revendications 10. ou 11 **caractérisée en ce que** les fibres kératiniques possèdent une hauteur de ton inférieure à 6.

## Claims

1. Fluorescent dye of formula **(I)** or **(Ia):** 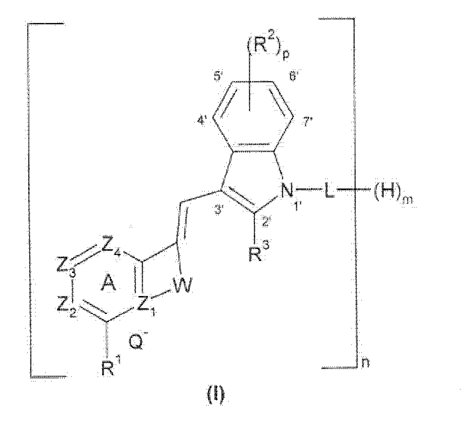 the or mineral acid salts, optical isomers and geometric isomers thereof, and the solvates such as hydrates: in which formula (I):
➢ **n** represents 1 or 2;
➢ **m** represents 0 or 1;
➢ **p** an integer between 0 ann 4 inclusive;
➢ **Q** represents an anionic counterion;
➢ **W** represents a (C₂-C₃)alkylene or (C₂-C₃)alkenylene chain optionally substituted with one or more (C₁-C₄)alkyl or aryl groups;
➢ **Z₁** represents a carbon atom or a nitrogen atom N⁺ which is quaternized;
➢ **Z₂, Z₃, Z₄**, which may be identical or different, represent a nitrogen atom, or a CR⁴ or N⁺R⁵ group; it being understood that only one quaternized nitrogen atom N⁺ or N⁺R⁵ may be present in the aromatic ring A;
**➢ R¹, R²** and **R⁴**, which may be identical or different, represent a halogen atom, a (di) (C₁-C₆) (alkyl)amino, cyans, hydroxyl, (posy) halo (C₁-C₆) alkyl such as trifluoromethyl, acyl(C₁-C₄)(alkyl) amino, (C₁-C₆)alkoxy, (C₁-C₅)alkylthio, (poly)hydroxy(C₂-C₆)alkoxy, (C₁-C₆)alkylcarbonyloxy, C₁-C₆alkoxycarbonyl, (C₁-C₆)alkylcarbonylamino, (di) (C₁-C₄) (alkyl) aminocarbonyl, (C₁-C₆)alkylsulfonyl (C₁-C₄) (alkyl) amino or (di) (C₁-C₄)(alkyl)aminosulfonyl group, or a (C₁-C₄)-alkyl group optionally substituted with a group chosen from (C₁-C₆)alkoxy, hydroxyl and (di) (C₁-C₆)-(alkyl)amino; or else, when p is an integer greater than or equal to 2, two contiguous radicals R² form, together with the carbon atom which bears them, a benzo ring;
**➢ R³** represents a hydrogen atom, an optionally substituted (C₁-C₆)alkyl alkyl group, a (di) (C₁-C₆)(alkyl)amino group, an aryl group, or a heterocycloalkyl group which is optionally substituted;
**➢ R⁵** represents an optionally substituted (C₁-C₆)alkyl or an aryl(C₁-C₅)alkyl; ;
**➢ L** represents a σ bond or an optionally substituted C₁-C₂₀ hydrocarbon-based chain, optionally interrupted i) with or divalent groups or combinations thereof chosen from: -N (R)-; -N⁺(R) (R°)-, An; -O-; -C(O)- and -S(O)₂-, with R, R° which may be identical or different, chosen from a hydrogen, and a (C₁-C₄) alkyl, hydroxy(C₁-C₄)alkyl or amino(C₁-C₆)alkyl radical, and An representing an anionic counterion, or ii) with a cationic heterocycle or cationic heteroaryl Het⁺, An, with An representing an anionic counterion and Het⁺ representing a saturated or unsaturated heterocycle comprising frog 5 to 10 members or a heteroaryl comprising from 5 to 10 members;
it being understood that, when n is 2, then m is zero, and when n is 1, then m is 1; and 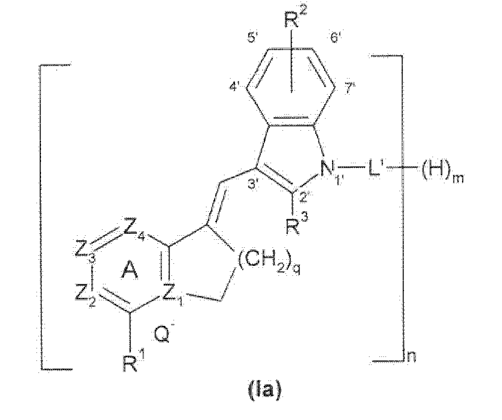 in which formula **(Ia):**
**➢ n, m, Q⁻** are as defined above;
➢ in which unit A:
a) either **Z₁** represents a nitrogen atom N⁺, and **Z₂, Z₃** and **Z₄** represent CR⁴, with R⁴ being a hydrogen atom or a (C₁-C₄) alkyl group, or **Z₃** and **Z₄** represent CH and **Z₂** represents a (C₁-C₆)alkylcarbonylamino or (di) (C₁-C₄) (alkyl)aminocarbonyl group;
b) or Z₂ represents a group N⁺R⁵, **Z₁** represents a carbon atom, and **Z₃** and **Z₄** represent a group CR⁴, with R⁴ being a hydrogen atom;
c) or **Z₄** represents a group N⁺R⁵, **Z₁** represents a carbon atom, and **Z₃** and **Z₂** represent a group CR⁴, with R⁴ being a hydrogen atom; and
d) or **Z₁** represents a nitrogen atom N⁺, **Z₂** and **Z₄** represent a group CR⁴ and **Z₃** represents a nitrogen atom;
➢ **q** is 1 or 2, particularly 1;
➢ **R¹** represents a hydrogen atom or a (C₁-C₆)alkyl group; in particular, R¹ represents a hydrogen atom;
➢ **R²** is absent, or represents a halogen atom, or a (C₁-C₃)alkoxy or (C₁-C₃)alkoxvcarbonyl group,
➢ **R³** represents a hydrogen atom, a (C₁-C₆)alkyl group such as methyl, a phenyl grout, a (di)(C₁-C₆) (alkyl)amino group such as dimethylamino, or a monocyclic, 5- to 7-membered heterocyloalkyl group comprising from 1 to 3 heteroatoms chosen from oxygen and nitrogen, optionally substituted with a (C₁-C₂)alkyl group such has piperazino;
➢ **R⁵** represents an optionally substituted (C₁-C₆)alkyl group, or an aryl(C₁-C₆)alkyl, in particular, R⁵ a (C₁-C₄)alkyl group such as methyl or methyl;
➢ **L'** represents a σ bond, a (C₂-C₆)alkylene chain such as ethylene -(CH₂)₂-, propylene -(GH₂)₃- or butylene -(CH₂)₄-, particularly ethylene; or a (C₁-C₆)alkyl group optionally substituted with, in particular, a (di) (C₁-C₆) (alkyl)aminocarbonyl group;
it being understood that, when n is 2, then m is zero, and when n is 1, then m is 1.

2. Fluorescent dye of formula **(I)** according to the preceding claim, in which m and n are 1.

3. Fluorescent dye of formula **(I)** according to Claim 1, in which n = 2 and m = 0.

4. Fluorescent dye of formula **(I)** according to any one of the preceding claims, comprising a unit A in which:
a) either **Z₁** represents a quaternized nitrogen atom N⁺, Z₂, **Z₃** and **Z₄** represent CR⁴, or **Z₃** and **Z₄** represent CH and **Z₂** represents a (C₁-C₆)alkylcarbonylamino or (di) (C₁-C₄) (alkyl)-group;
b) or **Z₂** represents a group N⁺R⁵ **Z₁** represents a carbon atom, and **Z₃** and **Z₄** reorec-ent at group CR⁴;
c) or **Z₄** represents a group N⁺-R⁵, **Z₁** represents a carbon atom, and **Z₃** and **Z₂** represent a group CR⁴ and
d) or **Z₁** represents a quaternized nitrogen atom N⁺, **Z₂** and **Z₄** represent a group CR⁴ and **Z**₃ represents a nitrogen atom.

5. Fluorescent dye of formula **(I)** or **(Ia)** according to any one of the preceding claims, chosen from the following dyes:
| | |
|---|---|
| 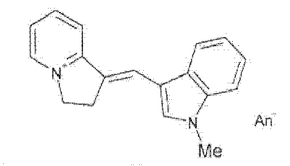 | 1 |
| 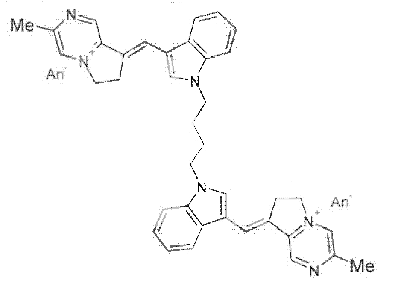 | 2 |
| | |
| 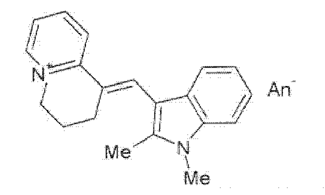 | 3 |
| 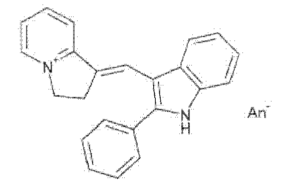 | 4 |
| 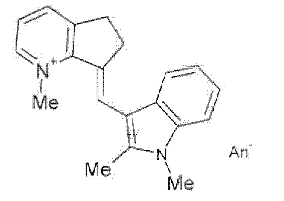 | 5 |
| 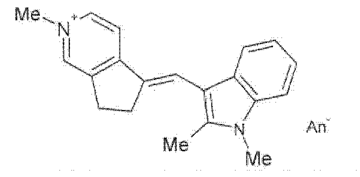 | 6 |
| 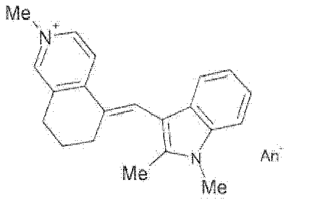 | 7 |
| 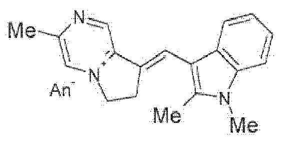 | |
| 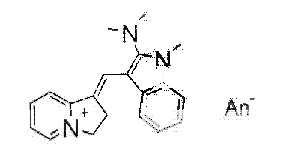 | 8 9 |
| 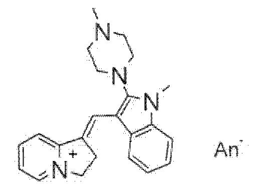 | 10 |
| 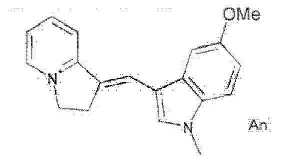 | 11 |
| 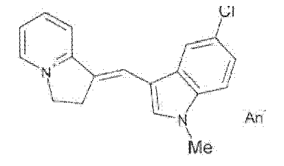 | 12 |
| 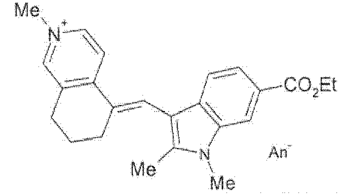 | 13 |
| 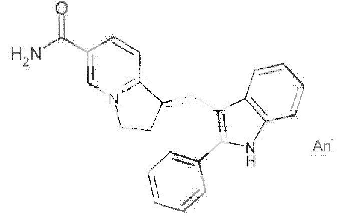 | 14 |
| 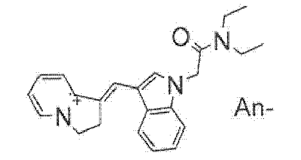 | 15 |
| 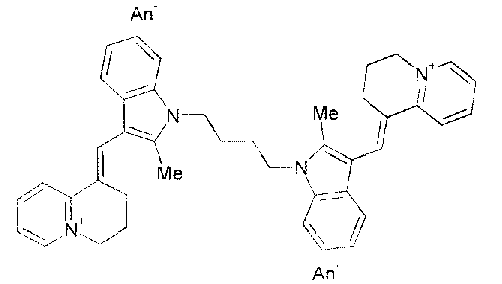 | 16 |
| 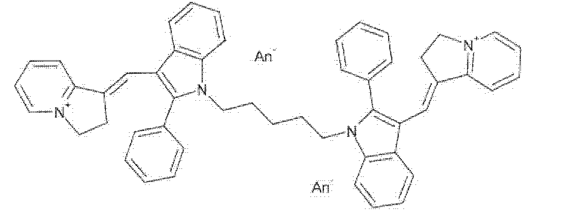 | 17 |
| 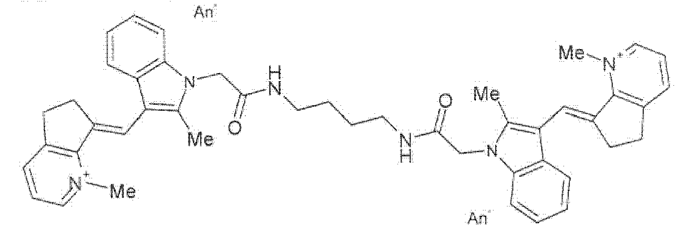 | 18 |
| 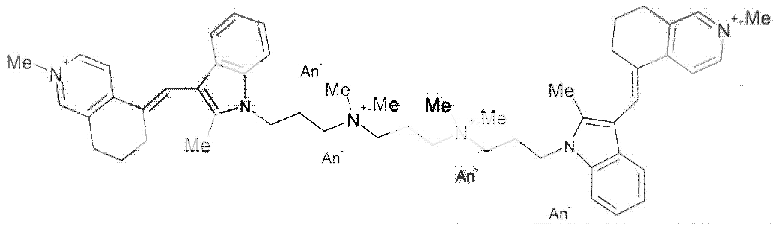 | 19 |
| 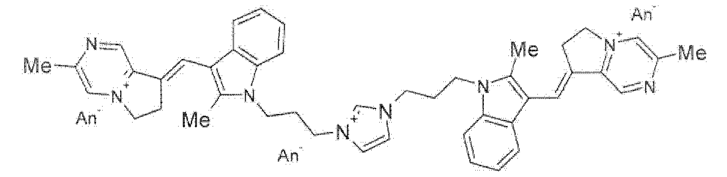 | 20 |
| 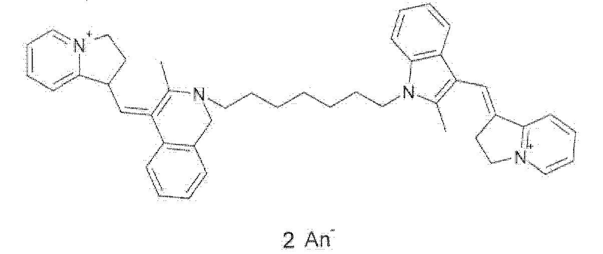 | 21 |
with An⁻, which may be identical or different, representing an anionic counterion.

6. Dye composition comprising, in a suitable cosmetic medium, a fluorescent dye of formula (I) or (Ia) as defined in any one of Claims 1 to 5.

7. Composition according to the preceding claim, in with the dye of formula (I) or (Ia) is present in an amount of between 0.001% and 50% by weight relative to the total weight of the composition.

8. Process for dyeing keratin materials, in which a cosmetic dye composition comprising at least one dye of formula (I) or (Ia) according to either one of Claims 6 and 7, is applied to the materials.

9. Process for dyeing keratin materials according to the preceding claim, **characterized in that** The keratin materials are dark keratin fibers having a tone height of less than or equal to 6.

10. Use of the dyes of formula (I) or (Ia) as defined in Claims 1 to 6, for dyeing dark human keratin fibers.

11. Use according to the preceding claim, for lightening dark keratin fibers.

12. Use according to Claim 10 or 11, **characterized in that** the keratin fibers have a tone height of less than 6.

## Patentansprüche

1. Fluoreszenzfarbstoff der Formel (I) oder (Ia): 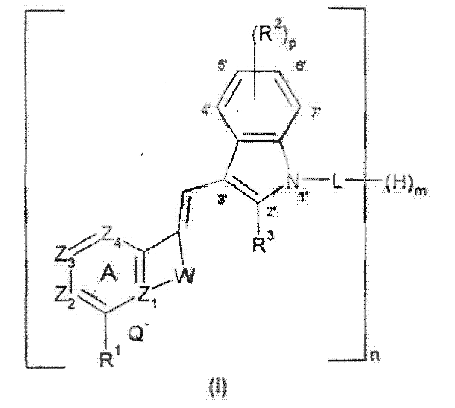 Salze davon mit einer organischen oder anorganischen Säure, optische Isomere davon, geometrische Isomere davon und Solvate wie Hydrate ;
wobei in der Formel (I) :
➢ n für 1 der 2 steht;
➢ m für 0 oder 1 steht;
➢ p für eine ganze Zahl zwischen 0 une 4 einschließlich steht;
➢ Q⁻ für ein anionisches steht;
➢ W für eine (C₂ - C₃) - Alkylen- oder (C₂-C₃)-Alkenylenkette, die gegebenenfalls durch eine oder mehrere (C₁-C₄)-Alkyl- oder Arylgruppen substituiert ist, steht;
➢ Z₁ für ein Kohlenstoffatom oder ein quaternisiertes Stickstoffatom N⁺ steht;
➢ Z₂, Z₃ und Z₄ gleich oder verschieden sind und für ein Stickstoffatom oder eine Gruppe CR⁴ oder N⁺R⁵ stehen, mit der Maßgabe, daß in dem aromatischen Ring A nur ein einziges quaternisiertes Stickstoffatom N⁺ oder N⁺R⁵ vorliegen kann;
➢ R¹, R² und R⁴ gleich oder verschieden sind und für ein Halogenatom, eine (Di) (C₁-C₆)(alkyl)amino-, cyano-, Hydroxy-, (Poly) halogen (C₁-C₆) - alkyl- wie Trifluormethyl-, Acyl(C₁-C₄) (alkyl)-amino-, (C₁-C₆)Alkoxy-, (C₁-C₆) Alkylthio-, (Poly) hydroxy (C₂-C₆) alkoxy-, (C₁-C₆)-carbonyloxy-, (C₁-C₆) Alkoxycarzonyl-, (C₁-C₆)-Alkylcarbonylamino-, (Di) (C₁-C₄) (alkyl)aminocarboxyl-, (C₁-C₆) Alkylsulfonyl(C₁-C₄) (alkyl) - amino- oder (Di) (C₁-C₄) (alkyle)aminosulfonylgruppe oder eine (C₁-C₆)Alkylgruppe, die gegebenenfalls durch eine unter (C₁-C₆)Alkoxy, Hydroxy und (Di) (C₁-C₆) (alkyl) amino ausgewählte Gruppe substituiert ist, stehen; oder dann, wenn p für eine ganze Zahl größer gleich 2 steht, zwei benachbarte Reste R² zusammen mit dem Kohlenstoffatom, an das sie gebunden sind, einen bilden;
➢ R³ für ein wasserstoffatom, eine gegebenenfalls substituierte (C₁-C₆)Alkylgruppe, eine (Di) (C₁-C₆) (alkyle) aminogruppe, eine Arylgruppe oder eine gegebenenfalls substituiert Heterocycloalkylgruppe steht;
➢ R⁵ für eine gegebenenfalls (C₁-C₆) Alkylgruppe ein Aryl(C₁-C₆)alkyl steht;
➢ L für eine σ-Bindung oder eine gegebenenfalls substituierte zweiweitige C₁-C₂₀-Kohlenwasserstoffkette steht, die gegebenenfalls i) durch eine oder mehrere zweiwertige Gruppen oder Kombinationen davon, die unter -N(R)-; -N⁺ (R) (R°) -, An⁻; -O-; -C(O)- und -S(O)₂-ausgewählt sind, wobei R und R° gleich oder verschieden sind und unter Wasserstoff und einem (C₁-C₄)Alkyl-, Hydroxy (C₁-C₄) alkyl- und Amino(C₁-C₄)alkylrest ausgewählt sind und An⁻ für ein anionisches Gegenion steht, oder ii) durch einen kationischen Heterocyclus oder ein kationisches Heteroaryl Het⁺, An⁻, wobei An⁻ für ein anionisches Gegenion steht und Het⁺ für einen gesättigten oder ungesättigten 5- bis 10-gliedrigen Heterocyclus oder ein 5- bis -10-gliedriges Heteroaryl steht, unterbrochen ist;
mit der Maßgabe, dann, n gleich 2 ist, m gleich null ist, und dann, wenn n gleich 1 ist, m gleich 1 ist; und 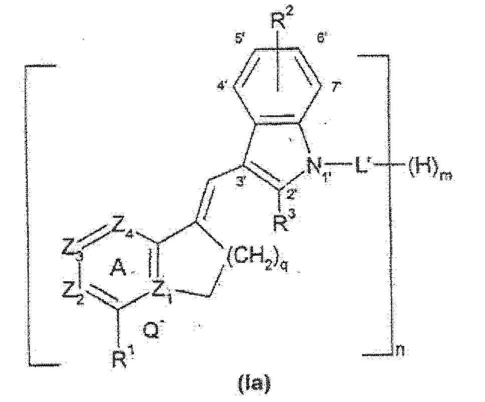 wobei in der Formel (Ia):
➢ n, m und Q⁻ die wie oben angegebene Bedeutung besitzen;
➢ wobei in der Einheit A:
a) entweder Z₁ für ein cruaternisiertes Stickstoffatom N⁺ steht und Z₂, Z₃ und Z₄ für CR⁴ stehen, wobei R⁴ für ein Wasserstoffatom oder eine (C₁-C₄)Alkylgruppe steht, oder Z₃ und Z₄ für CH stehen und Z₂ für eine (C₁-C₆) Alkylcarbonylamino- oder (Di) (C₁-C₄) (alkyl) aminocarbonylgruppe steht;
b) oder Z₂ für eine Gruppe N⁺R⁵ steht, Z₁ für ein Kohlenstoffatom, steht und Z₃ und Z₄ für eine Gruppe CR⁴ stehen, wobei R⁴ für ein Wasserstoffatom steht;
c) oder Z₄ für eine Gruppe N⁺R⁵ steht, Z₁ für ein Kohlenstoffatom steht und Z₃ und Z₂ für eine Gruppe CR⁴ stehen, wobei R⁴ für ein Wassersteht;
d) oder Z₁ für ein quaternisiertes Stickstoffatom N+ steht, Z₂ und z₄ für eine Gruppe CR⁴ stehen und Z₃ für Stickstoffatom steht;
➢ q gleich 1 2, insbesondere 1, ist;
➢ R¹ für ein Wasserstofatom oder eine (C₁-C₆)-Allylgruppe steht und R¹ insbesondere für sein Wasserstoffatom steht;
➢ R² fehlt für ein Halogenatom oder eine (C₁-C₃) Alkoxy- oder (C₁-C₃)Alkoxycarbonylgruppe steht;
➢ R³ für ein Wasserstoffatom, eine (C₁-C₅)Alkylgruppe wie Ethyl, eine Phenylgruppe, eine (Di-) (C₁-C₆) (alkyl) aminogruppe wie Dimethylamino, oder eine monocyclische 5-7-gliedrige Heterocycloalkylgruppe mit 1 bis 3 unter Sauerstoff und Stickstoff ausgewählten Heteroatomen, die gegebenenfalls durch eine (C₁-C₂)-Alkylgruppe substituiert ist, wie Piperazino, steht;
➢ R⁵ für eine gegebenenfalls substituierte (C₁-C₆) Alkylgruppe oder ein Aryl (C₁-C₆)alkyl steht und R⁵ insbesondere für eine (C₁-C₄)Alkylgruppe wie Methyl oder Ethyl steht;
L' für eine σ-Bindung, eine (C₂-C₆)Alkylenkette wie Ethylen -(CH₂)₂-, Propylen (CH₂)₃- oder Butylen -(CH₂)₄-, insbesondere Ethylen; oder eine (C₁-C₆) Alkylgruppe, die gegebenenfalls durch insbesondere eine (Di) (C₁-C₆) (alkyl) aminocarbonylgruppe substituiert ist, steht;
mit der Maßgabe, daß darin, wenn n gleich 2 ist, m gleich null ist, und dann, n gleich 1 ist, m gleich 1 ist.

2. Fluoreszenzfarbstoff der Formel (I) nach dem vorhergehenden Anspruch, worin m und n gleich 1 sind.

3. Fluoreszenzfarbstoff der Formel (I) nach Anspruch 1, worin n=2 m=0.

4. Fluoreszenzfarbstoff nach einem der vorhergehenden Ansprüche mit einer Einheit A, in der:
a) entweder Z₁ für ein cruaternisiertes Stickstoffatom N⁺ steht und Z₂, Z₃ und Z₄ für CR⁴ stehen oder Z₃ und Z₄ für CH stehen und Z₂ für eine C₁-C₆) Alkylecarbonylamino- oder (Di)(C₁-C₄)(alkyl)-aminocarbonylgruppe steht,
b) oder Z₂ für eine Gruppe N⁺R⁵ steht, Z₁ für ein Kohlenstoffatom steht und Z₃ und Z₄ für eine Gruppe CR⁴ stehen;
c) oder Z₄ für eine Gruppe N⁺R⁵ steht, Z₁ für ein Kohlenstoffatom steht und Z₃ und Z₂ für eine Gruppe CR⁴ stehen;
d) oder Z₁ für ein quaternisiertes Stickstoffatom N⁺ steht, Z₂ und Z₄ für eine Gruppe CR⁴ stehen und Z₃ für Stickstoffatom steht.

5. der Formel (I) oder (Ia) nach einem der vorhergehenden Ansprüche, ausgewählt unter den Farbstoffen:
| | |
|---|---|
| 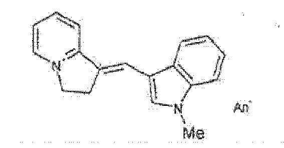 | 1 |
| 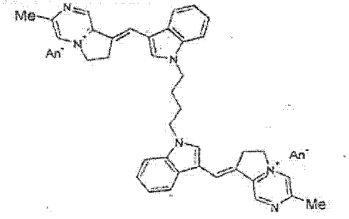 | 2 |
| 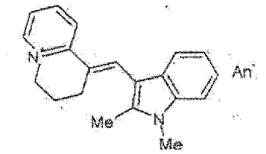 | 3 |
| 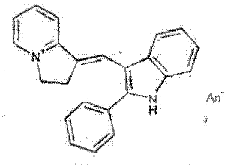 | 4 |
| 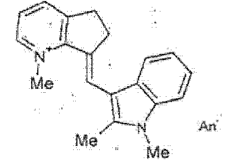 | 5 |
| | |
| 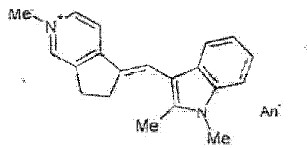 | 6 |
| 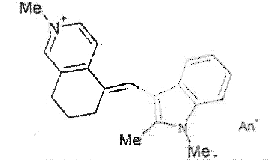 | |
| 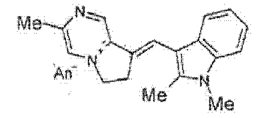 | 8 |
| 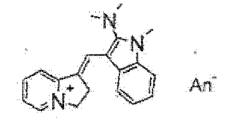 | 9 |
| 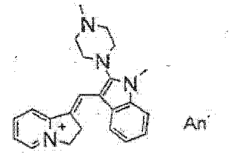 | 10 |
| 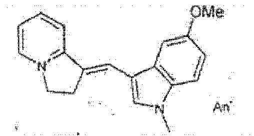 | 11 |
| 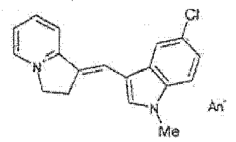 | 12 |
| 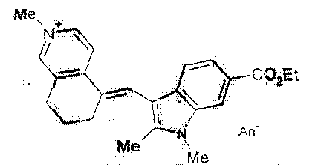 | 13 |
| | |
| 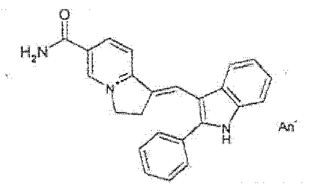 | 14 |
| 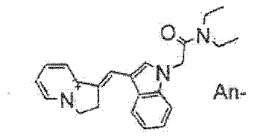 | 15 |
| 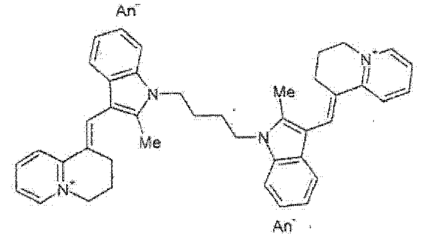 | 16 |
| 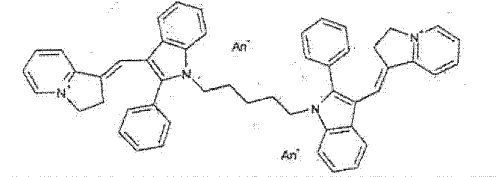 | 17 |
| 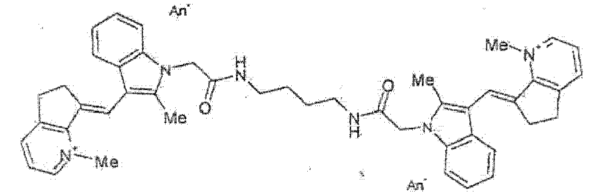 | 18 |
| 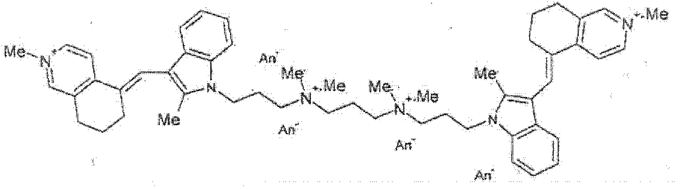 | 19 |
| | |
| 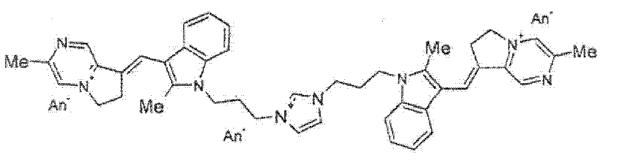 | 20 |
| 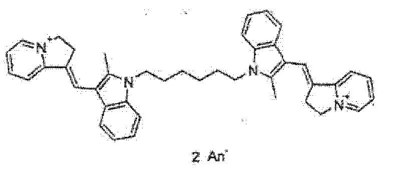 | 21 |
wobei die Gruppen An- gleich verschieden sind und für ein anionisches Gegenion stehen.

6. Färbezusammensetzung, die in geeigneten kosmetischen Medium einen Fluoreszenzfarbstoff der Formel (I) oder (Ia) gemäß einem der Ansprüche 1 bis 5 umfaßt.

7. Zusaromensetzung nach dem vorhergehenden Anspruch, in der der Farbstoff der Formel (I) oder (Ia) in einer Menge zwischen 0,001 und 50 Gew.-%, bezogen auf das Gesamtgewicht der Zusammensetzung, vorliegt.

8. Verfahren zum Färben von Keratinmaterialien, bei dem man auf die Materialien eine kosmetische Färbezusammensetzung, die mindestens einen stoff der Formel (I) oder (Ia) umfaßt, gemäß einem der ansprüche 6 oder aufbringt.

9. Verfahren zum Färben von Keratinmaterialien nach dem vorhergehenden Anspruch, **dadurch gekennzeichnet, daß** es sich bei den Keratinmaterialien um dunkle Keratinfasern mit einer Tonhöhe kleiner gleich 6 handelt.

10. Verwendung der Farbstoffe der Formel (I) oder (Ia) gemäß einem der Ansprüche 1 bis 6 zum Färben von dunklen menschlichen Keratinfasern.

11. Verwendung nach dem vorhergehenden Anspruch zur Aufhellung von dunklen Keratinfasern.

12. Verwendung nach den Ansprüchen 10 oder 11, **dadurch** gekenntzeichnet, daß die Keratinfasern eine Tonhöhe kleiner 6 aufweisen.
